# EUROPÄISCHE PATENTSCHRIFT

(11) **EP 0 707 576 B1**
(45) Veröffentlichungstag und Bekanntmachung des Hinweises auf die Patenterteilung: **03.03.1999**
(21) Anmeldenummer: 94919657.0
(22) Anmeldetag: 22.06.1994
(51) Int. Cl.: C07D 307/94, C07D 487/04

(54) **SUBSTITUIERTE ARYL-KETOENOLHETEROCYCLEN**
SUBSTITUTED ARYL-KETO-ENOLIC HETEROCYCLES
HETEROCYCLES ARYL-CETOENOLIQUES SUBSTITUES

(30) Priorität: 05.07.1993 DE 4322273; 20.04.1994 DE 4413669
(43) Veröffentlichungstag der Anmeldung: 24.04.1996
(73) Patentinhaber: BAYER AG, 51368 Leverkusen (DE)
(72) Erfinder: BACHMANN, Jürgen, D-51373 Leverkusen (DE); BRETSCHNEIDER, Thomas, D-53721 Siegburg (DE); FISCHER, Reiner, D-40789 Monheim (DE); KRÜGER, Bernd-Wieland, D-51467 Bergisch Gladbach (DE); SANTEL, Hans-Joachim, D-51371 Leverkusen (DE); DOLLINGER, Markus, D-51381 Leverkusen (DE); ERDELEN, Christoph, D-42799 Leichlingen (DE); WACHENDORFF-NEUMANN, Ulrike, D-40789 Monheim (DE)
(74) Vertreter: Schumacher, Günter, Dr.
(86) Internationale Anmeldenummer: EP9402042
(87) Internationale Veröffentlichungsnummer: WO9501971

(56) Entgegenhaltungen:
- EP-A- 0 355 599
- EP-A- 0 508 126
- EP-A- 0 528 156
- DE-A- 4 014 420
- DE-A- 4 213 026

## Beschreibung

Die vorliegende Erfindung betrifft neue substituierte Aryl-ketoenolheterocyclen, mehrere Verfahren zu ihrer Herstellung und ihre Verwendung als Schädlingsbekämpfungsmittel.

Es ist bekannt, daß bestimmte substituierte Δ³-Dihydrofuran-2-on-Derivate herbizide Eigenschaften besitzen (vgl. DE-A 4 014 420). Die Synthese der als Ausgangsverbindungen verwendeten Tetronsäurederivate (wie z.B. 3-(2-Methyl-phenyl)-4-hydroxy-5-(4-fluorphenyl)Δ³-dihydrofuranon-(2)) ist ebenfalls in DE-A 4 014 420 beschrieben. Ähnlich strukturierte Verbindungen ohne Angabe einer insektiziden und/oder akariziden Wirksamkeit sind aus der Publikation Campbell et al. J. Chem. Soc., Perkin Trans. 1 1985, (8) 1567-76 bekannt.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985 1095) synthetisiert. Eine biologische Wirksamkeit dieser Verbindungen wurde nicht beschrieben.

In EP-A 0 262 399 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A 355 599 und EP 415 211), substituierte bicyclische 3-Aryl -pyrrolidin-2,4-dion-Derivate (EP 501 129) sowie substituierte mono-cyclische 3-Aryl-pyrrolidin-2,4-dion-Derivate (EP-A 377 893, EP 442 077 und EP 497 127).

In DE 42 13 026 werden 4-Alkoxy- bzw. 4-(substituierte) Amino-3-arylpyrrolinon-Derivate und ihre Verwendung als Herbizide und Fungizide beschrieben.

Weiterhin bekannt sind polycyclische 3-Arylpyrrolidin-2,4-dion-Derivate (EP 442 073), 1-H-3-Arylpyrrolidin-dion-Derivate (EP 456 063 und EP 521 334) sowie substituierte bicyclische 3-Arylpyrrolidin-dion-Derivate (EP 501 129).

Weiterhin bekannt sind Tetronsäurederivate mit fungiziden, herbiziden, akariziden und insektiziden Eigenschaften (EP 528 156).

4-Arylpyrazolidin-dion-Derivate mit herbiziden, akariziden und insektiziden Eigenschaften sind in WO 92/16510 und EP 508 126 beschrieben.

Es wurden nun neue substituierte Aryl-ketoenolheterocyclen der Formel (I) 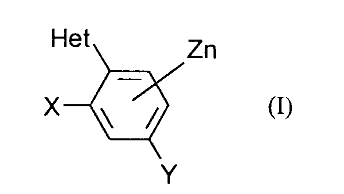 gefunden,
in welcher
- X: für Alkyl, Halogen oder Alkoxy steht,
- Y: für Wasserstoff, Alkyl, Halogen, Alkoxy oder Halogenalkyl steht,
- Z: für Alkyl, Halogen oder Alkoxy steht,
- n: für eine Zahl 0, 1, 2 oder 3 steht,
- Het: für eine heterocyclische Gruppe aus der Reihe 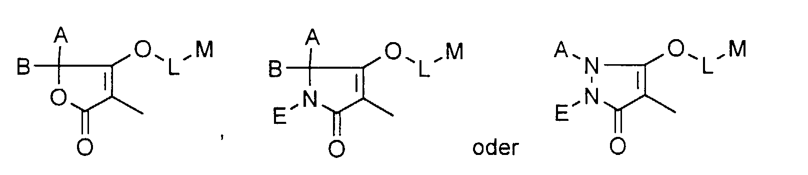
steht,
- A und B: gleich oder verschieden sein können und für Wasserstoff, jeweils gegebenenfalls durch Halogen substituiertes Alkyl, Alkenyl, Alkoxyalkyl, Alkylthioalkyl, gegebenenfalls durch mindestens ein Heteroatom unterbrochenes Cycloalkyl oder jeweils gegebenenfalls durch Halogen, Alkyl, Halogenalkyl, Alkoxy, Halogenalkoxy oder Nitro substituiertes Aryl, Aralkyl oder Hetaryl stehen,
oder worin
- A und B: gemeinsam mit dem Kohlenstoffatom, an das sie gebunden sind, einen gesättigten oder ungesättigten, gegebenenfalls durch mindestens ein Heteroatom unterbrochenen und gegebenenfalls substituierten Cyclus bilden,
- E: für Wasserstoff, jeweils gegebenenfalls durch Halogen substituiertes Alkyl, Alkenyl, Alkoxyalkyl, Alkylthioalkyl, gegebenenfalls durch mindestens ein Heteroatom unterbrochenes Cycloalkyl oder jeweils gegebenenfalls durch Halogen, Alkyl, Halogenalkyl, Alkoxy, Halogenalkoxy oder Nitro substituiertes Aryl, Aralkyl oder Hetaryl steht,
oder worin
- A und E: gemeinsam mit den Atomen, an die sie gebunden sind, einen gesättigten oder ungesättigten, gegebenenfalls durch mindestens ein Heteroatom unterbrochenen und gegebenenfalls substituierten Cyclus, Bicyclus oder Tricyclus bilden,
- L: für eine Alkandiylgruppe steht,
- M: für eine der folgenden Gruppierungen steht: oder 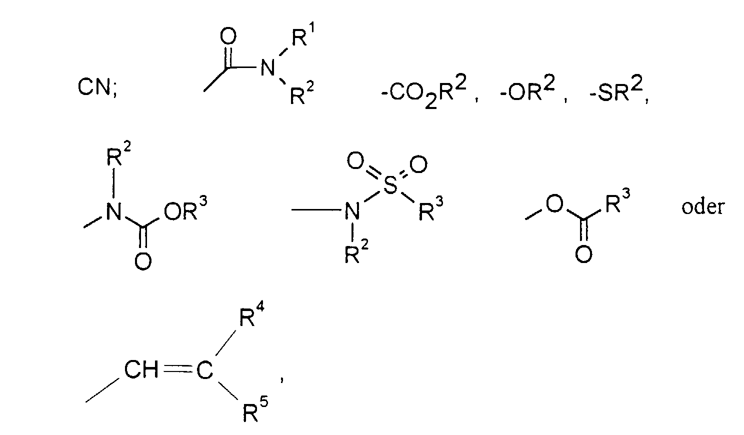
wobei
- R¹: für Wasserstoff oder Alkyl steht,
- R²: für jeweils gegebenenfalls substituiertes Alkyl, Alkenyl, Alkinyl, Cycloalkyl, Alkoxyalkyl, Alkylthioalkyl, Aryl oder Aralkyl steht,
- R³: für gegebenenfalls substituiertes Alkyl, Aryl oder Aralkyl steht,
- R⁴: für Wasserstoff, Halogen, jeweils gegebenenfalls substituiertes Alkyl oder Phenyl steht,
- R⁵: für Wasserstoff, Halogen oder gegebenenfalls substituiertes Alkyl steht,
sowie die enantiomerenreinen Formen von Verbindungen der Formel (I).

Die Verbindungen der Formel (I) können in Abhängigkeit der Substituenten als geometrische und/oder optische Isomere oder Isomerengemische unterschiedlicher Zusammensetzung vorliegen. Sowohl die reinen Isomeren als auch die Isomerengemische, deren Herstellung und Verwendung sowie diese enthaltende Mittel sind Bestandteile der beanspruchten Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Weiterhin wurde gefunden, daß die neuen Verbindungen der Formel (I) eine sehr gute Wirksamkeit als Schädlingsbekämpfungsmittel, vorzugsweise als Arthropodizide, Fungizide und Herbizide aufweisen.

Weiterhin wurde gefunden, daß man die neuen substituierten Aryl-ketoenolheterocyclen der Formel (Ia) 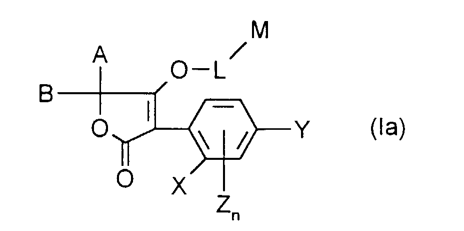 in welcher
A, B, L, M, X, Y, Z und n die oben angegebene Bedeutung haben,
erhält,
wenn man
(A) Verbindungen der Formel (IIa) 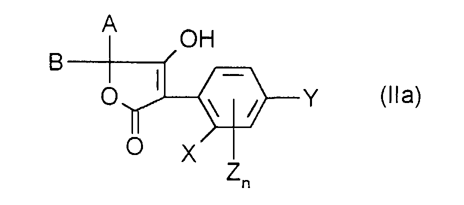 in welcher
   A, B, X, Y, Z und n die oben angegebene Bedeutung haben,
   mit Verbindungen der Formel (III)

   G-L-M (III)

   in welcher
   L und M die oben angegebene Bedeutung haben
   und
   - G: für eine Abgangsgruppe, wie Halogen, Sulfonylalkyl oder Sulfonylaryl steht,
   in Gegenwart eines Verdünnungsmittels und in Gegenwart einer Base umsetzt.
(B) Weiterhin erhält man Verbindungen der Formel (Ib) 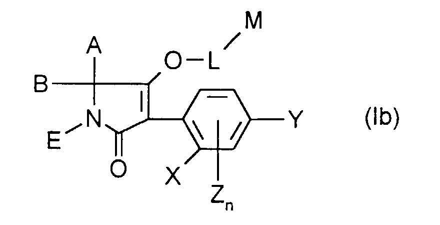 in welcher
   A, B, E, L, M, X, Y, Z und n die oben angegebene Bedeutung haben,
   wenn man Verbindungen der Formel (IIb) 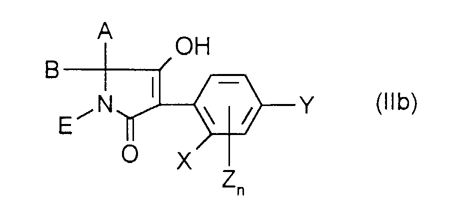 in welcher
   A, B, E, X, Y, Z und n die oben angegebene Bedeutung haben,
   mit Verbindungen der Formel (III)

   G-L-M (III)

   in welcher
   L und M die oben angegebene Bedeutung haben
   und
   - G: für eine Abgangsgruppe, wie Halogen, Sulfonylalkyl oder Sulfonylaryl steht,
   in Gegenwart eines Verdünnungsmittels und in Gegenwart einer Base umsetzt.
(C) Außerdem erhält man Verbindungen der Formel (Ic) 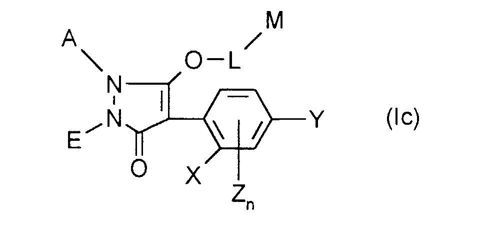 in welcher
   A, E, L, M, X, Y, Z und n die oben angegebene Bedeutung haben,
   wenn man Verbindungen der Formel (IIc) 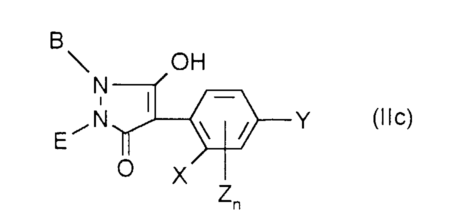 in welcher
   B, E, X, Y, Z und n die oben angegebene Bedeutung haben,
   mit Verbindungen der Formel (III)

   G-L-M (III)

   in welcher
   L und M die oben angegebene Bedeutung haben
   und
   - G: für eine Abgangsgruppe, wie Halogen, Sulfonylalkyl oder Sulfonylaryl steht,
   in Gegenwart eines Verdünnungsmittels und in Gegenwart einer Base umsetzt.

Überraschenderweise zeigen die neuen substituierten Aryl-ketoenolheterocyclen (Ia), (Ib) und (Ic) gute akarizide, insektizide und herbizide Eigenschaften.

Die erfindungsgemäßen Verbindungen sind durch die Formel (I) allgemein definiert.
- X: steht bevorzugt für C₁-C₆-Alkyl, Halogen oder C₁-C₆-Alkoxy.
- Y: steht bevorzugt für Wasserstoff, C₁-C₆-Alkyl, Halogen, C₁-C₆-Alkoxy oder C₁-C₃-Halogenalkyl.
- Z: steht bevorzugt für C₁-C₆-Alkyl, Halogen oder C₁-C₆-Alkoxy.
- n: steht bevorzugt für eine Zahl von 0 bis 3.
- Het: steht bevorzugt für eine heterocyclische Gruppe aus der Reihe 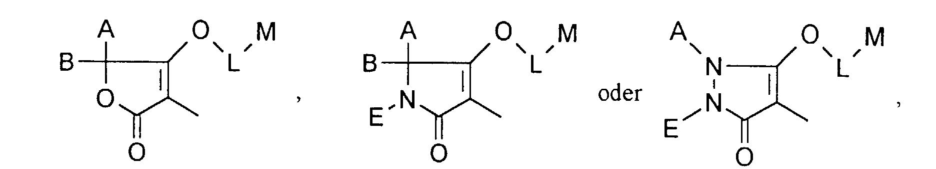
worin
- A und B: gleich oder verschieden sind und für Wasserstoff oder jeweils gegebenenfalls durch Halogen substituiertes geradkettiges oder verzweigtes C₁-C₁₂-Alkyl, C₃-C₈-Alkenyl, C₁-C₁₀-Alkoxy-C₂-C₈-alkyl, C₁-C₈-Polyalkoxy-C₂-C₈-alkyl, C₁-C₁₀-Alkylthio-C₂-C₈-alkyl, Cycloalkyl mit 3 bis 8 Ringatomen, das durch Sauerstoff und/oder Schwefel unterbrochen sein kann oder jeweils gegebenenfalls durch Halogen, C₁-C₆-Alkyl, C₁-C₆-Haloalkyl, C₁-C₆-Alkoxy, C₁-C₆-Halogenalkoxy oder Nitro substituiertes Phenyl, Pyrimidyl, Imidazolyl, Pyrazolyl, Triazolyl, Indolyl, Thiazolyl oder Phenyl-C₁-C₆-alkyl steht,
oder worin
- A und B: gemeinsam mit dem Kohlenstoffatom, an das sie gebunden sind, einen gesättigten oder ungesättigten, gegebenenfalls durch Sauerstoff und/oder Schwefel unterbrochenen und gegebenenfalls durch Halogen, C₁-C₆-Alkyl, C₁-C₆-Alkoxy, C₁-C₄-Halogenalkyl, C₁-C₄-Halogenalkoxy, C₁-C₄-Alkylthio oder gegebenenfalls durch Halogen, C₁-C₆-Alkyl, C₁-C₆-Alkoxy substituiertes Phenyl substituierten 3- bis 8-gliedrigen Ring bilden,
- E: für Wasserstoff oder jeweils gegebenenfalls durch Halogen substituiertes geradkettiges oder verzweigtes C₁-C₁₂-Alkyl, C₃-C₈-Alkenyl, C₁-C₁₀-Alkoxy-C₂-C₈-alkyl, C₁-C₈-Polyalkoxy-C₂-C₈-alkyl, C₁-C₁₀-Alkylthio-C₂-C₈-alkyl, Cycloalkyl mit 3 bis 8 Ringatomen, das durch Sauerstoff und/oder Schwefel unterbrochen sein kann oder jeweils gegebenenfalls durch Halogen, C₁-C₆-Alkyl, C₁-C₆-Halogenalkyl, C₁-C₆-Alkoxy, C₁-C₆-Halogenalkoxy oder Nitro substituiertes Phenyl, Pyrimidyl, Imidazolyl, Pyrazolyl, Triazolyl, Indolyl, Thiazolyl oder Phenyl-C₁-C₆-alkyl steht,
oder worin
- A und E: gemeinsam mit den Atomen, an die sie gebunden sind, einen gesättigten oder ungesättigten, gegebenenfalls durch Sauerstoff und/oder Schwefel unterbrochenen und gegebenenfalls durch Halogen, C₁-C₆-Alkyl, C₁-C₆-Alkoxy, C₁-C₄-Halogenalkyl, C₁-C₄-Halogenalkoxy, C₁-C₄-Alkylthio oder gegebenenfalls durch Halogen, C₁-C₆-Alkyl, C₁-C₆-Alkoxy substituiertes Phenyl substituierten 3- bis 8-gliedrigen Ring bilden,
oder für den Fall, daß Het in Formel (I) für einen Pyrazolinonring steht
- A und E: zusammen mit den beiden Stickstoffatomen des Pyrazolinrings für eine gegebenenfalls einfach oder mehrfach, gleich oder verschieden durch C₁-C₆-Alkyl, C₁-C₆-Halogenalkyl oder Halogen substituierte Gruppierung der nachfolgend aufgeführten Formeln 1 bis 4 stehen 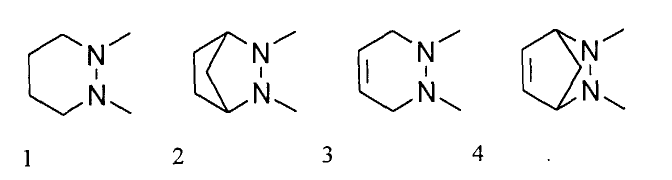
- L: steht bevorzugt für eine Alkandiylgruppe mit 1 bis 6 Kohlenstoffatomen.
- M: steht bevorzugt für eine der folgenden Gruppierungen: 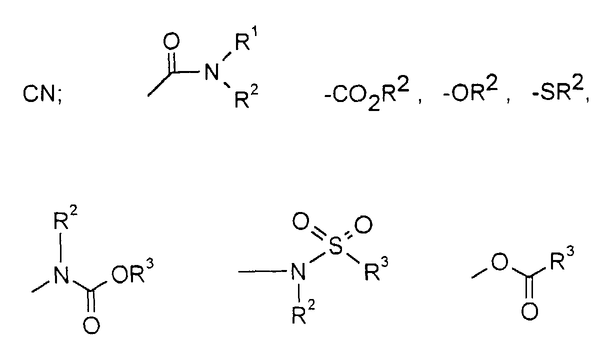 oder 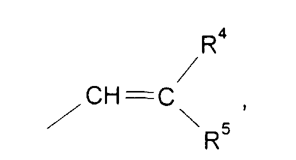
- R¹: steht bevorzugt für Wasserstoff oder C₁-C₁₂-Alkyl.
- R²: steht bevorzugt für jeweils gegebenenfalls durch Halogen substituiertes C₁-C₁₂-Alkyl, C₃-C₈-Alkenyl, C₃-C₈-Alkinyl, C₃-C₈-Cycloalkyl, C₁-C₈-Alkoxy-C₂-C₈-alkyl, C₁-C₈-Alkylthio-C₂-C₈-alkyl, für jeweils gegebenenfalls durch Halogen, Nitro, Cyano, C₁-C₆-Alkoxy, C₁-C₆-Halogenalkoxy, C₁-C₆-Alkylthio, C₁-C₆-Halogenakylthio, C₁-C₆-Alkyl oder C₁-C₆-Halogenalkyl substituiertes Phenyl oder Benzyl.
- R³: steht bevorzugt für gegebenenfalls durch Halogen substituiertes C₁-C₁₂-Alkyl oder für jeweils durch Halogen, C₁-C₆-Alkyl oder C₁-C₆-Alkoxy substituiertes Phenyl oder Benzyl.
- R⁴: steht bevorzugt für Wasserstoff, Halogen, gegebenenfalls durch Halogen substituiertes C₁-C₆-Alkyl oder jeweils durch Halogen, C₁-C₆-Alkyl, C₁-C₆-Alkoxyl, C₁-C₄-Haloalkyl, C₁-C₄-Haloalkoxy, Cyano oder Nitro substituiertes Phenyl.
- R⁵: steht bevorzugt für Wasserstoff, Halogen oder gegebenenfalls durch Halogen substituiertes C₁-C₆-Alkyl.
- X: steht besonders bevorzugt für C₁-C₆-Alkyl, Fluor, Chlor, Brom oder C₁-C₆-Alkoxy.
- Y: steht besonders bevorzugt für Wasserstoff, C₁-C₆-Alkyl, Fluor, Chlor, Brom, C₁-C₆-Alkoxy oder C₁-C₂-Halogenalkyl.
- Z: steht besonders bevorzugt für C₁-C₄-Alkyl, Fluor, Chlor, Brom oder C₁-C₄-Alkoxy.
- n: steht besonders bevorzugt für eine Zahl von 0 bis 2.
- Het: steht besonders bevorzugt für eine heterocyclische Gruppe der Reihe 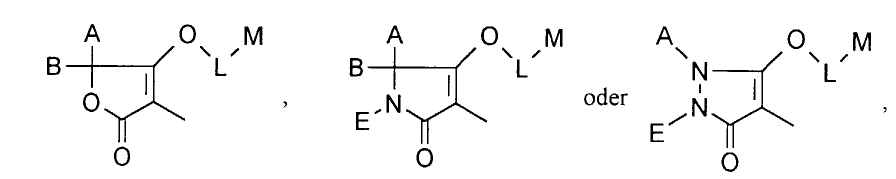
worin
- A und B: gleich oder verschieden sind und für Wasserstoff, jeweils gegebenenfalls durch Halogen substituiertes geradkettiges oder verzweigtes C₁-C₁₀-Alkyl, C₃-C₆-Alkenyl, C₁-C₈-Alkoxy-C₂-C₆-alkyl, C₁-C₆-Polyalkoxy-C₂-C₂-alkyl, C₁-C₈-Alkylthio-C₂-C₆-alkyl, Cycloalkyl mit 3 bis 7 Ringatomen, das durch 1-2 Sauerstoff- und/oder Schwefelatome unterbrochen sein kann oder jeweils gegebenenfalls durch Halogen, C₁-C₄-Alkyl, C₁-C₄-Halogenalkyl, C₁-C₄-Alkoxy oder Nitro substituiertes Phenyl, Pyridinyl, Imidazolyl, Pyrazolyl, Triazolyl, Indolyl, Thiazolyl oder Phenyl-C₁-C₄-alkyl stehen,
oder worin
- A und B: gemeinsam mit dem Kohlenstoffatom, an das sie gebunden sind, einen gesättigten oder ungesättigten, gegebenenfalls durch Sauerstoff und/oder Schwefel unterbrochenen und gegebenenfalls durch Halogen, C₁-C₅-Alkyl, C₁-C₅-Alkoxy, C₁-C₃-Halogenalkyl, C₁-C₄-Halogenalkoxy, C₁-C₃-Alkylthio oder gegebenenfalls durch Halogen, C₁-C₄-Alkyl, C₁-C₄-Alkoxy substituiertes Phenyl substituierten 3- bis 8-gliedrigen Ring bilden,
- E: für Wasserstoff oder jeweils gegebenenfalls durch Halogen substituiertes geradkettiges oder verzweigtes C₁-C₁₀-Alkyl, C₃-C₆-Alkenyl, C₁-C₈-Alkoxy-C₂-C₆-alkyl, C₁-C₆-Polyalkoxy-C₂-C₆-alkyl, C₁-C₈-Alkylthio-C₂-C₆-alkyl, Cycloalkyl mit 3 bis 7 Ringatomen, das durch 1-2 Sauerstoff-und/oder Schwefelatome unterbrochen sein kann oder jeweils gegebenenfalls durch Halogen, C₁-C₄-Alkyl, C₁-C₄-Halogenalkyl, C₁-C₄-Alkoxy oder Nitro substituiertes Phenyl, Pyridinyl, Imidazolyl, Pyrazolyl, Triazolyl, Indolyl, Thiazolyl oder Phenyl-C₁-C₄-alkyl steht,
oder worin
- A und E: gemeinsam mit den Atomen, an die sie gebunden sind, einen gesättigten oder ungesättigten, gegebenenfalls durch Sauerstoff und/oder Schwefel unterbrochenen und gegebenenfalls durch Halogen, C₁-C₅-Alkyl, C₁-C₅-Alkoxy, C₁-C₃-Halogenalkyl, C₁-C₄-Halogenalkoxy, C₁-C₃-Alkylthio oder gegebenenfalls durch Halogen, C₁-C₄-Alkyl, C₁-C₄-Alkoxy substituiertes Phenyl substituierten 3- bis 8-gliedrigen Ring bilden,
oder für den Fall, daß in Formel (I) Het für einen Pyrazolinonring steht
- A und E: zusammen mit den beiden Stickstoffatomen des Pyrazolinrings für eine gegebenenfalls einfach oder mehrfach, gleich oder verschieden durch C₁-C₄-Alkyl, C₁-C₄-Halogenalkyl, Fluor oder Chlor substituierte Gruppierung der nachfolgend aufgeführten Formeln 1 oder 2 stehen, 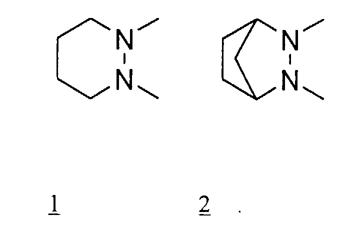
- L: steht besonders bevorzugt für eine Alkandiylgruppe mit 1 bis 4 Kohlenstoffatomen.
- M: steht besonders bevorzugt für eine der folgenden Gruppierungen: 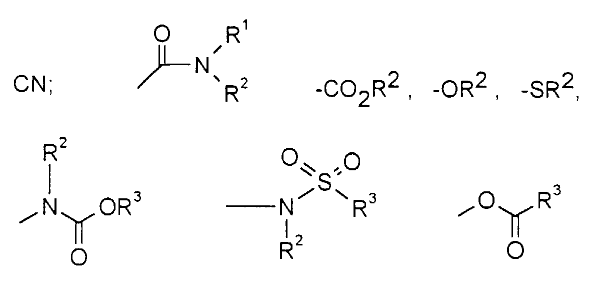 oder 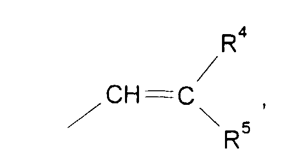
- R¹: steht besonders bevorzugt für Wasserstoff oder C₁-C₁₀-Alkyl.
- R²: steht besonders bevorzugt für jeweils gegebenenfalls durch Fluor, Chlor, Brom substituiertes C₁-C₁₀-Alkyl, C₃-C₆-Alkenyl, C₃-C₆-Alkinyl, C₃-C₈-Cycloalkyl, C₁-C₆-Alkoxy-C₂-C₆-alkyl, C₁-C₆-Alkylthio-C₂-C₆-alkyl, für jeweils gegebenenfalls durch Fluor, Chlor, Brom, Nitro, Cyano, C₁-C₄-Alkylthio, C₁-C₄-Alkoxy, C₁-C₄-Halogenalkoxy, C₁-C₄-Halogenalkylthio, C₁-C₄-Alkyl oder C₁-C₄-Halogenalkyl substituiertes Phenyl oder Benzyl.
- R³: steht besonders bevorzugt für gegebenenfalls durch Fluor, Chlor, Brom substituiertes C₁-C₁₀-Alkyl oder für jeweils durch Fluor, Chlor, C₁-C₄-Alkyl oder C₁-C₄-Alkoxy substituiertes Phenyl oder Benzyl.
- R⁴: steht besonders bevorzugt für Wasserstoff, Fluor, Chlor, Brom, gegebenenfalls durch Fluor oder Chlor substituiertes C₁-C₅-Alkyl oder durch Fluor, Chlor, Brom, C₁-C₄-Alkyl, C₁-C₄-Alkoxy, C₁-C₂-Haloalkyl, C₁-C₂-Haloalkoxy, Cyano oder Nitro substituiertes Phenyl.
- R⁵: steht besonders bevorzugt für Wasserstoff, Fluor, Chlor, Brom oder gegebenenfalls durch Fluor oder Chlor substituiertes C₁-C₄-Alkyl.
- X: steht ganz besonders bevorzugt für Methyl, Ethyl, Propyl, i-Propyl, Fluor, Chlor, Brom oder Methoxy.
- Y: steht ganz besonders bevorzugt für Wasserstoff, Methyl, Ethyl, Propyl, i-Propyl, Butyl, i-Butyl, tert.-Butyl, Fluor, Chlor, Brom, Methoxy, Ethoxy und Trifluormethyl.
- Z: steht ganz besonders bevorzugt für Methyl, Ethyl, Propyl, i-Propyl, Butyl, i-Butyl, tert.-Butyl, Fluor, Chlor, Brom, Methoxy und Ethoxy.
- n: steht ganz besonders bevorzugt für 1.
- Het: steht ganz besonders bevorzugt für eine heterocyclische Gruppe aus der Reihe 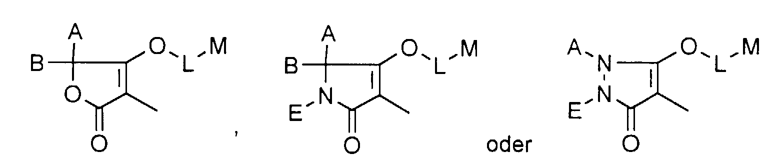
worin
- A und B: gleich oder verschieden sind und für Wasserstoff, jeweils gegebenenfalls durch Fluor oder Chlor substituiertes geradkettiges oder verzweigtes C₁-C₈-Alkyl, C₃-C₄-Alkenyl, C₁-C₆-Alkoxy-C₂-C₄-alkyl, C₁-C₂-Polyalkoxy-C₂-C₄-alkyl, C₁-C₆-Alkylthio-C₂-C₂-alkyl, Cycloalkyl mit 3 bis 6 Ringatomen, das durch 1-2 Sauerstoff- und /oder Schwefelatome unterbrochen sein kann oder jeweils gegebenenfalls durch Fluor, Chlor, Methyl, Ethyl, Propyl, iso-Propyl, Methoxy, Ethoxy, Trifluormethyl oder Nitro-substituiertes Phenyl, Pyridinyl, Imidazolyl, Indolyl oder Phenyl-C₁-C₃-alkyl stehen,
oder worin
- A und B: gemeinsam mit dem Kohlenstoffatom, an das sie gebunden sind, einen gesättigten oder ungesättigten, gegebenenfalls durch Sauerstoff und/oder Schwefel unterbrochenen und gegebenenfalls durch Fluor, Chlor, C₁-C₄-Alkyl, C₁-C₄-Alkoxy, Trifluormethyl, C₁-C₂-Alkylthio oder gegebenenfalls durch Fluor, Chlor, Methyl, Methoxy substituiertes Phenyl substituierten 3-bis 8-gliedrigen Ring bilden,
- E: für Wasserstoff oder jeweils gegebenenfalls durch Fluor oder Chlor substituiertes geradkettiges oder verzweigtes C₁-C₈-Alkyl, C₃-C₄-Alkenyl, C₁-C₆-Alkoxy-C₂-C₄-alkyl, C₁-C₄-Polyalkoxy-C₂-C₄-alkyl, C₁-C₆-Alkylthio-C₂-C₄-alkyl, Cycloalkyl mit 3 bis 6 Ringatomen, das durch 1-2 Sauerstoff- und/oder Schwefelatome unterbrochen sein kann oder gegebenenfalls durch Fluor, Chlor, Methyl, Ethyl, Propyl, iso-Propyl, Methoxy, Ethoxy, Trifluormethyl oder Nitro substituiertes Phenyl, Pyridinyl, Imidazolyl, Indolyl oder Phenyl-C₁-C₃-alkyl steht,
oder worin
- A und E: gemeinsam mit den Atomen, an die sie gebunden sind, einen gesättigten oder ungesättigten, gegebenenfalls durch Sauerstoff und/oder Schwefel unterbrochenen und gegebenenfalls durch Fluor, Chlor, C₁-C₄-Alkyl, C₁-C₄-Alkoxy, Trifluormethyl, C₁-C₂-Alkylthio oder gegebenenfalls durch Fluor, Chlor, Methyl, Methoxy substituiertes Phenyl substituierten 3- bis 8-gliedrigen Ring bilden,
oder für den Fall, daß in Formel (I) Het für einen Pyrazolinonring steht
- A und E: zusammen mit den beiden Stickstoffatomen des Pyrazolinrings für eine gegebenenfalls einfach bis dreifach, gleich oder verschieden durch Methyl, Fluor oder Chlor substituierte Gruppierung der nachfolgend aufgeführten Formeln 1 oder 2 stehen 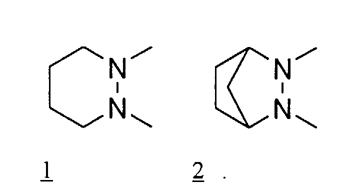
- L: steht ganz besonders bevorzugt für eine der folgenden Gruppierungen. 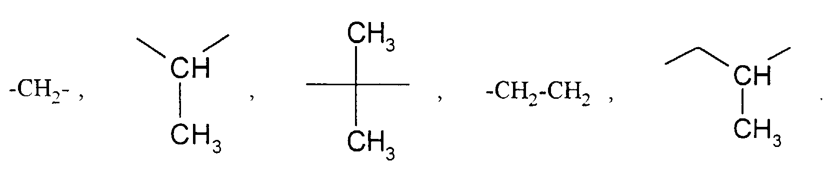
- M: steht ganz besonders bevorzugt für eine der folgenden Gruppierungen. 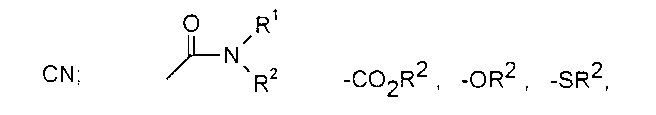 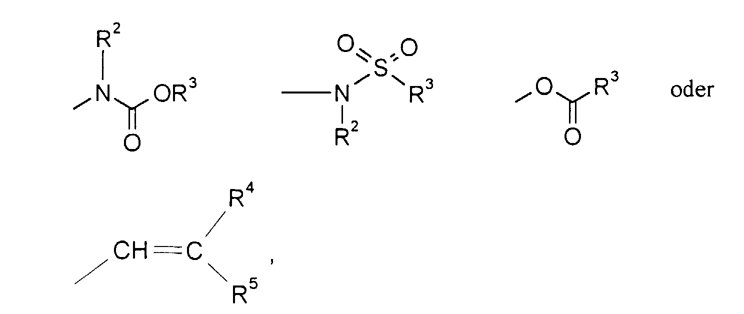
- R¹: steht ganz besonders bevorzugt für Wasserstoff oder C₁-C₈-Alkyl.
- R²: steht ganz besonders bevorzugt für jeweils gegebenenfalls durch Fluor, Chlor, Brom substituiertes C₁-C₈-Alkyl, C₃-C₄-Alkenyl, C₃-C₄-Alkinyl, Cyclopropyl, Cyclopentyl, Cyclohexyl, C₁-C₆-Alkoxy-C₂-C₄-alkyl, C₁-C₆-Alkylthio-C₂-C₄-alkyl, für jeweils gegebenenfalls durch Fluor, Chlor, Brom, Nitro, Cyano, Methylthio, Ethylthio, Methoxy, Ethoxy, Trifluormethylthio, Trifluormethoxy, Methyl, Ethyl, Trifluormethyl substituiertes Phenyl oder Benzyl.
- R³: steht ganz besonders bevorzugt für jeweils gegebenenfalls durch Fluor, Chlor, Brom substituiertes C₁-C₈-Alkyl oder durch Fluor, Chlor, C₁-C₂-Alkyl oder C₁-C₂-Alkoxy substituiertes Phenyl oder Benzyl.
- R⁴: steht ganz besonders bevorzugt für Wasserstoff, Fluor, Chlor, gegebenenfalls durch Fluor oder Chlor substituiertes C₁-C₄-Alkyl oder durch Fluor, Chlor, Methyl, Ethyl, Methoxy, Ethoxy, Trifluormethyl, Difluormethoxy, Trifluormethoxy, Cyano oder Nitro substituiertes Phenyl.
- R⁵: steht ganz besonders bevorzugt für Wasserstoff, Fluor, Chlor oder gegebenenfalls durch Fluor oder Chlor substituiertes Methyl, Ethyl, Propyl oder Isopropyl.

Dabei sind die enantiomerenreinen Formen von Verbindungen der Formel (I) jeweils eingeschlossen.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechen.

Erfindungsgemäß bevorzugt werden die Verbindungen der allgemeinen Formel (I), in welchen eine Kombination der vorstehend als bevorzugt (vorzugsweise) aufgeführten Bedeutungen vorliegt.

Erfindungsgemäß besonders bevorzugt werden die Verbindungen der allgemeinen Formel (I), in welchen eine Kombination der vorstehend als besonders bevorzugt aufgeführten Bedeutungen vorliegt.

Erfindungsgemäß ganz besonders bevorzugt werden die Verbindungen der allgemeinen Formel (I) verwendet, in welchen eine Kombination dieser vorstehend als ganz besonders bevorzugt aufgeführten Bedeutungen vorliegt.

Verwendet man gemäß Verfahren (A) 3-(2,4-Dichlorphenyl)-4-hydroxy-5-methyl-Δ³-furan-2-on und (Chlormethyl)-ethylether, so kann der Verlauf des erfindungsgemäßen Verfahrens durch folgendes Reaktionsschema wiedergegeben werden:

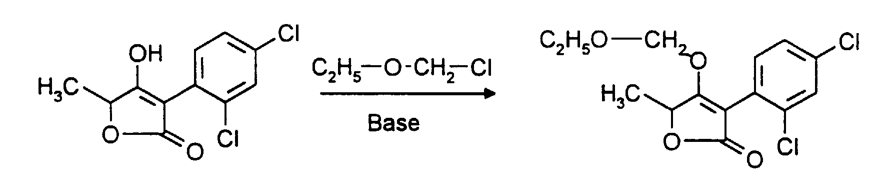

Verwendet man gemäß Verfahren (B) 3-(2,6-Dichlorphenyl)-4-hydroxy-1-isopropyl-Δ³-pyrrolin-2-on und N-Chlormethyl-N-methyl-carbaminsäure-ethylester, so kann der Verlauf des erfindungsgemäßen Verfahrens durch folgendes Reaktionsschema wiedergegebenen werden.

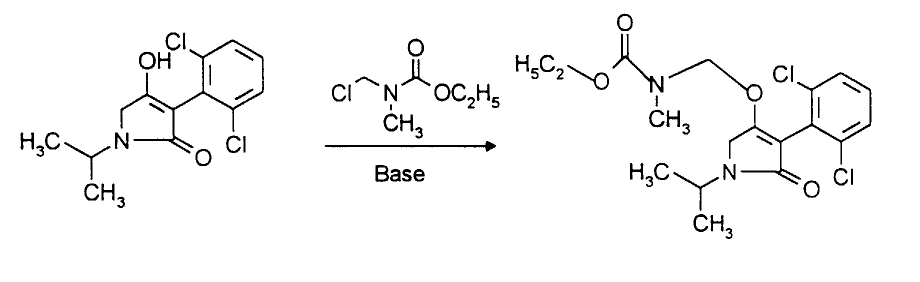

Verwendet man gemäß Verfahren (C) 3-(2,4,6-Trimethylphenyl)-5-hydroxy-1,2-tetramethylen-Δ⁴-pyrazolin-3-on und (Chlormethyl)-phenylether, so läßt sich das erfindungsgemäße Verfahren durch folgendes Reaktionsschema wiedergegeben:

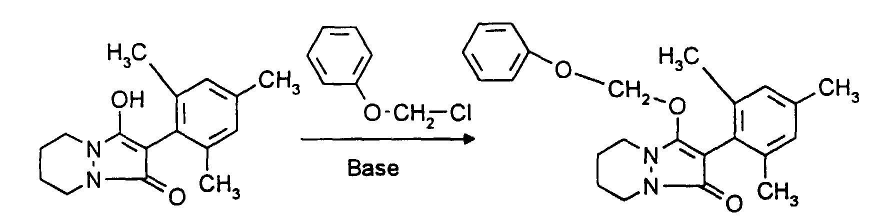

Die bei dem Verfahren (A) als Ausgangsstoffe benötigten Verbindungen der Formel (IIa) 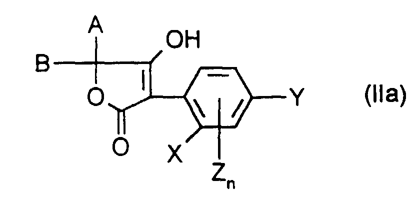 in welcher
A, , X, Y, Z und n die oben angegebene Bedeutung haben, sind bekannt (EP 528 156).

Die bei dem Verfahren (B) als Ausgangsstoffe benötigten Verbindungen der Formel (IIb) 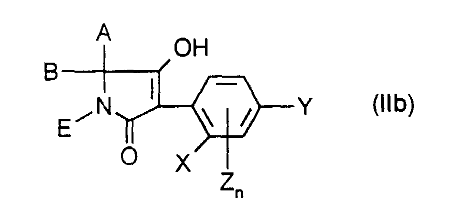 in welcher
A, B, E, X, Y, Z und n die oben angegebene Bedeutung haben, sind bekannt (vgl. beispielsweise EP 355 999, EP 377 893, EP 415 211, EP 442 073, EP 456 063, EP 497 127, EP 501 129).

Die bei dem Verfahren (C) als Ausgangsstoffe benötigten Verbindungen der . Formel (IIc) 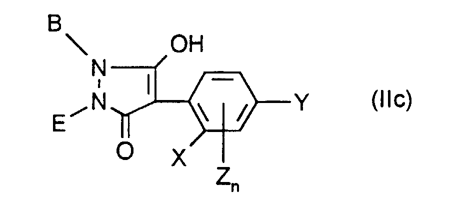 in welcher
E, B, X, Y, Z und n die oben angegebene Bedeutung haben,
sind bekannt (vgl. beispielsweise WO 92/16510 und EP 508 126).

Die zur Durchführung der erfindungsgemäßen Verfahren (A), (B) und (C) weiterhin als Ausgangsverbindungen benötigten Verbindungen der Formel (III) sind allgemein bekannte Verbindungen der organischen Chemie.

Als Verdünnungsmittel können für die Durchführung der erfindungsgemäßen Verfahren (A), (B) und (C) alle gegenüber diesen Verbindungen inerten Solventien eingesetzt werden. Vorzugsweise verwendbar sind Kohlenwasserstoffe, wie Benzin, Benzol, Toluol, Xylol und Tetralin; ferner Halogenkohlenwasserstoffe, wie Methylenchlorid, Chloroform, Tetrachlorkohlenstoff, Chlorbenzol und o-Dichlorbenzol; Ether, wie Diethylether, Tetrahydrofuran und Dioxan; Nitrile, wie Acetonitril oder Propionitril; ferner polare, gegenüber den Verbindungen der Formel (III) inerte Lösungsmittel, wie Dimethylsulfoxid, Dimethylformamid, Sulfolan oder N-Methylpyrrolidon.

Als Basen können bei der Durchführung der erfindungsgemäßen Verfahren (A), (B) und (C) alle üblichen Säureakzeptoren eingesetzt werden. Vorzugsweise verwendbar sind Alkalimetall- und Erdalkalimetall-oxide, -hydroxide und - carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464^{*)} oder TDA 1^{***)} eingesetzt werden können. Ferner sind Alkalimetall- und Erdalkalimetall-hydride, wie Natriumamid, Natriumhydrid und Calciumhydrid, und außerdem auch Alkalimetallalkoholate, wie Natriummethylat, Natriumethylat und Kalium-tert.-butylat einsetzbar. Weiterhin tertiäre Amine, wie Triethylamin, Pyridin, DABCO, DBU, DBA, Hünig-Base und N,N-Dimethylanilin.
^{*)} Adogen 464 = Methyltrialkyl(C₈.C₁₀)ammoniumchlorid
^{***)} TDA 1 =Tris-(methoxyethoxyethyl)-amin

Die Reaktionstemperaturen können bei der Durchführung der erfindungsgemäßen Verfahren (A), (B) und (C) innerhalb eines größeren Bereiches variiert werden. Im allgemeinen arbeitet man bei Temperaturen zwischen -20°C und 180°C, vorzugsweise zwischen 0°C und 130°C.

Bei der Durchführung der erfindungsgemäßen Verfahren (A), (B) und (C) setzt man die Reaktionskomponenten (IIa) und (III) oder (IIb) und (III) oder (IIc) und (III) und die Base im allgemeinen in etwa äquimolaren Mengen ein. Es ist jedoch auch möglich, die eine oder andere Komponente in einem größeren Überschuß (bis zu 3 Mol) zu verwenden.

Die erfindungsgemäßen Verfahren (A), (B) und (C) werden im allgemeinen unter Normaldruck durchgeführt. Die Aufarbeitung erfolgt nach üblichen Methoden.

Die Wirkstoffe eignen sich bei guter Pflanzenverträglichkeit zur Bekämpfung von tierischen Schädlingen, vorzugsweise Arthropoden und Nematoden; insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria . mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Milben, wie beispielsweise gegen die gemeine Spinnmilbe oder die Bohnenspinnmilbe (Tetranychus urticae) oder gegen die Obstbaummilbe (Panonychus ulmi) einsetzen.

Die erfindungsgemäßen Wirkstoffe zeigen außerdem auch eine fungizide Wirksamkeit, eine Wirksamkeit gegen Pyricularia oryzae und Wirkung gegen Oomyceten wie beispielsweise Phythophtora und Plasmopara).

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich sehr gut zur selektiven Bekämpfung monokotyler Unkräuter in dikotylen Kulturen im Vorauflaufverfahren. Sie können beispielsweise in Soja mit sehr gutem Erfolg zur Bekämpfung von Schadgräsern eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mit:5 teln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Aryl-sulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Genannt seien die folgenden Verbindungen:
Acrinathrin, Alphamethrin, Betacyfluthrin, Bifenthrin, Brofenprox, Cis-Resmethrin, Clocythrin, Cycloprothrin, Cyfluthrin, Cyhalothrin, Cypermethrin, Deltamethrin, Esfenvalerate, Etofenprox, Fenpropathrin, Fenvalerate, Flucythrinate, Fluvalinate, Lambda-Cyhalothrin, Permethrin, Pyresmethrin, Pyrethrum, Silafluofen, Tralomethrin, Zeta-methrin, Alanycarb, Bendiocarb, Benfuracarb, Bufencarb, Butocarboxim, Carbaryl, Cartap, Ethiofencarb, Fenobucarb, Fenoxycarb, Isoprocarb, Methiocarb, Methomyl, Metolcarb, Oxamyl, Pirimicarb, Promecarb, Propoxur, Terbam, Thiodicarb, Thiofanox, Trimethacarb, XMC, Xylylcarb, Acephate, Azinphos A, Azinphos M, Bromophos A, Cadusafos, Carbophenothion, Chlorfenvinphos, Chlormephos, Chlorpyrifos, Chlorpyrifos M, Cyanophos. Demeton M, Demeton-S-methyl, Demeton S, Diazinon, Dichlorvos, Dicliphos, Dichlorfenthion, Dicrotophos, Dimethoate, Dimethylvinphos, Dioxathion, Disulfoton, Edifenphos, Ethion, Etrimphos, Fenitrothion, Fenthion, Fonophos, Formothion, Heptenophos, Iprobenfos, Isazophos, Isoxathion, Phorate, Malathion, Mecarbam, Mervinphos, Mesulfenphos, Methacrifos, Methamidophos, Naled, Omethoate, Oxydemeton M, Oxydeprofos, Parathion A, Parathion M, Phenthoate, Phorate, Phosalone, Phosmet, Phosphamdon, Phoxim, Pirimiphos A, Pirimiphos M, Propaphos, Prothiophos, Prothoate, Pyraclophos, Pyridaphenthion, Quinalphos, Salithion, Sebufos, Sulfotep, Sulprofos, Tetrachlorvinphos, Temephos, Thiomethon, Thionazin, Trichlorfon, Triazophos, Vamidothion, Buprofezin, Chlorfluazuron, Diflubenzuron, Flucycloxuron, Flufenoxuron, Hexaflumuron, Pyriproxifen, Tebufenozide, Teflubenzuron, Triflumuron, Imidacloprid, Nitenpyram, N-[(6-Chloro-3-pyridinyl)methyl]-N'-cyano-N-methyl-ethanimidamid (NI-25), Abamectin, Amitrazin, Avermectin, Azadirachtin, Bensultap, Bacillus thuringiensis, Cyromazine, Diafenthiuron, Emamectin, Ethofenprox, Fenpyrad, Fipronil, Flufenprox, Lufenuron, Metaldehyd, Milbemectin, Pymetrozine, Tebufenpyrad, Triazuron, Aldicarb, Bendiocarb, Benfuracarb, Carbofuran, Carbosulfan, Chlorethoxyfos, Cloethocarb, Disulfoton, Ethophrophos, Etrimphos, Fenamiphos, Fipronil, Fonofos, Fosthiazate, Furathiocarb, HCH, Isazophos, Isofenphos, Methiocarb, Monocrotophos, Nitenpyram, Oxamyl, Phorate, Phoxim, Prothiofos, Pyrachlofos, Sebufos, Silafluofen, Tebupirimphos, Tefluthrin, Terbufos, Thiodicarb, Thiafenox, Azocyclotin, Butylpyridaben, Clofentezine, Cyhexatin, Diafenthiuron, Diethion, Emamectin, Fenazaquin, Fenbutatin Oxide, Fenothiocarb, Fenpropathrin, Fenpyrad, Fenpyroximate, Fluazinam, Fluazuron, Flucycloxuron, Flufenoxuron, Fluvalinate, Fubfenprox, Hexythiazox, Ivemectin, Methidathion, Monocrotophos, Moxidectin, Naled, Phosalone, Profenofos, Pyraclofos, Pyridaben, Pyrimidifen, Tebufenpyrad, Thuringiensin, Triarathene sowie 4-Bromo-2-(4-chlorophenyl)-1-(ethoxymethyl)-5-(trifluoromethyl)-1H-pyrrole-3 -carbonitril (AC 303630).

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

### Herstellungsbeispiele:

### Beispiel Ia-1

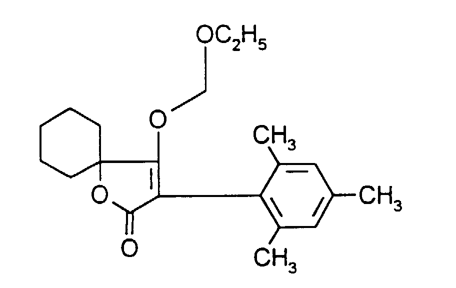

Zu 4,3 g (15 mmol) 3-(2,4,6-Trimethylphenyl)-4-hydroxy-5,5-pentamethylen-Δ³-dihydrofuran-2-on in 60 ml absolutem Dichlormethan, 1,67 g (16,5 mmol) Triethylamin und einer Spatelspitze DMAP werden unter Stickstoffatmosphäre bei 0°C bis 10°C 1,47 (15,5 mmol) Chlormethylethylether in 15 ml absolutem Dichlormethan zugetropft. Der Reaktionsansatz wird bei Raumtemperatur etwa 20 Stunden gerührt, anschließend nacheinander mit 10%iger Citronensäure, Natriumhydrogencarbonat- und Natriumchloridlösung gewaschen, die organische Phase mit Natriumsulfat getrocknet und das Lösungsmittel abgezogen. Das Rohprodukt wird an einer Kieselgelsäule (Laufmittel: Chloroform/Essigester 3:1) weiter gereinigt.

Man erhält 2,47 g (53% der Theorie) 3-(2,4,6-Trimethylphenyl)-4-(ethoxymethyloxy)-5,5-pentamethylen-Δ³-dihydrofuran-2-on vom Schmelzpunkt Fp. 102°C.

In Analogie zu Beispiel 1 wurden die Verbindungen der folgenden Herstellungsbeispiele der Formel (Ia) synthetisiert:

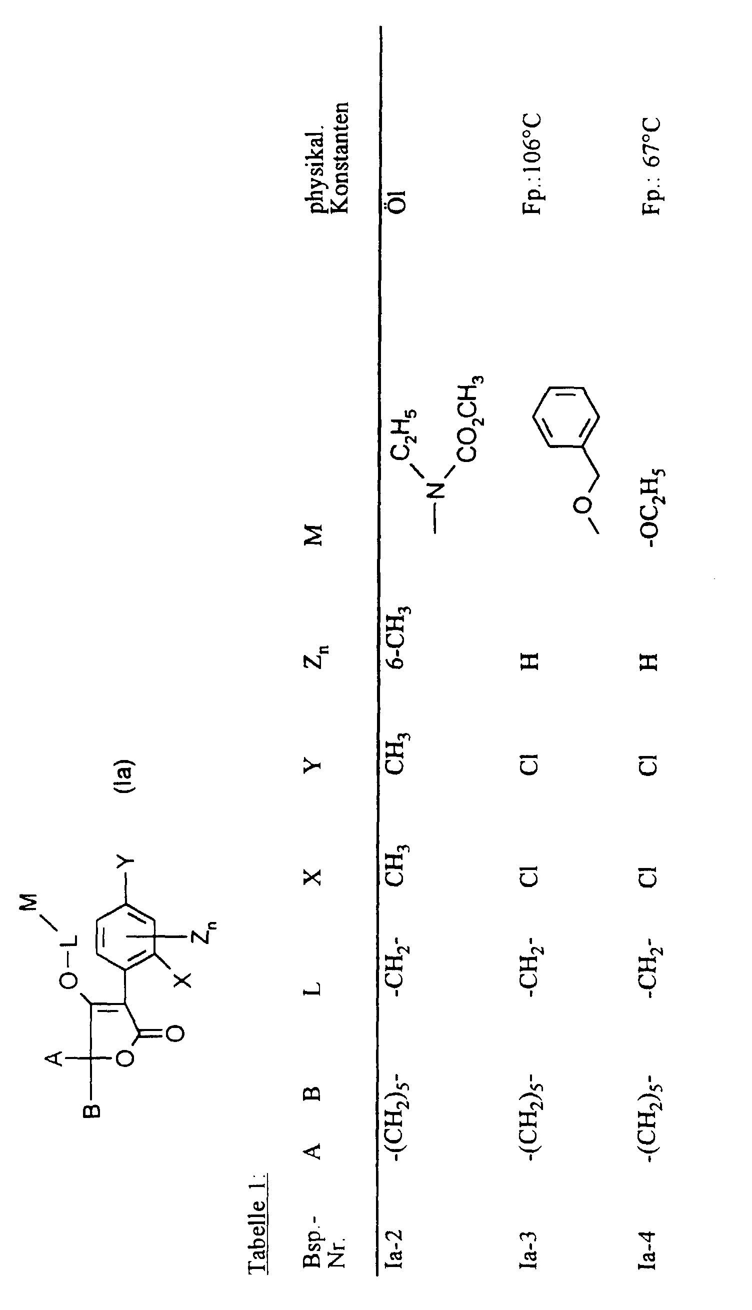

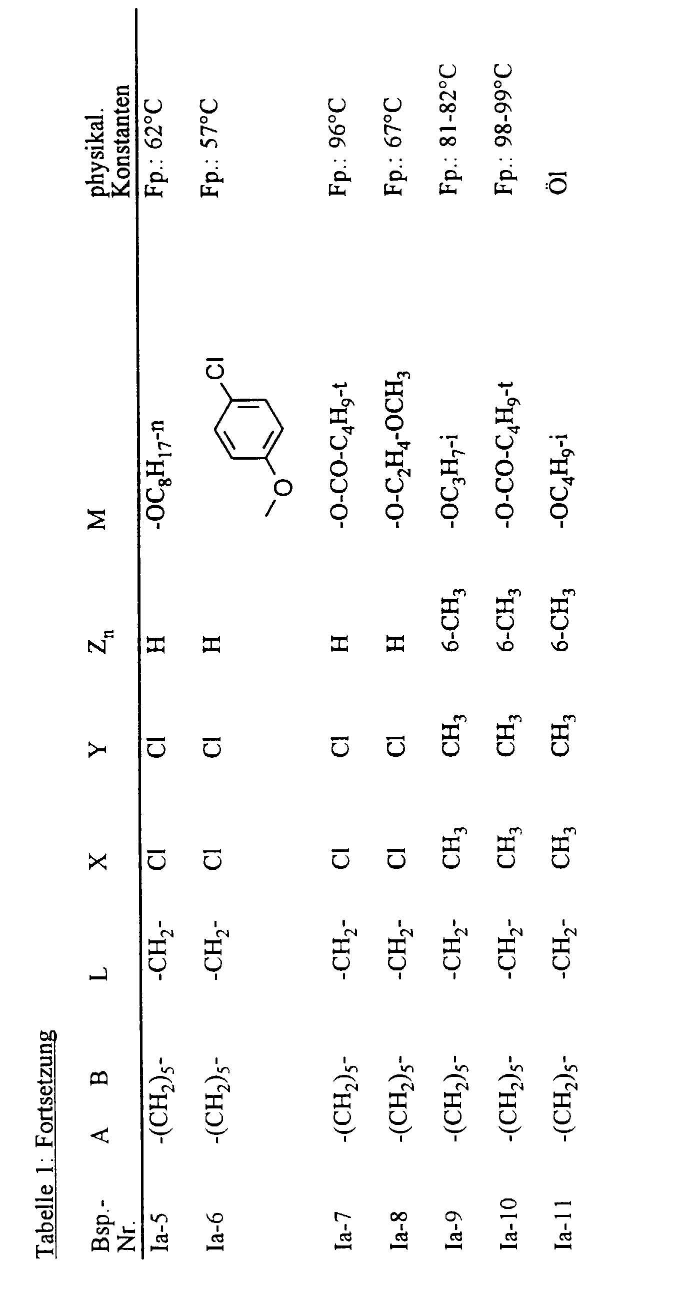

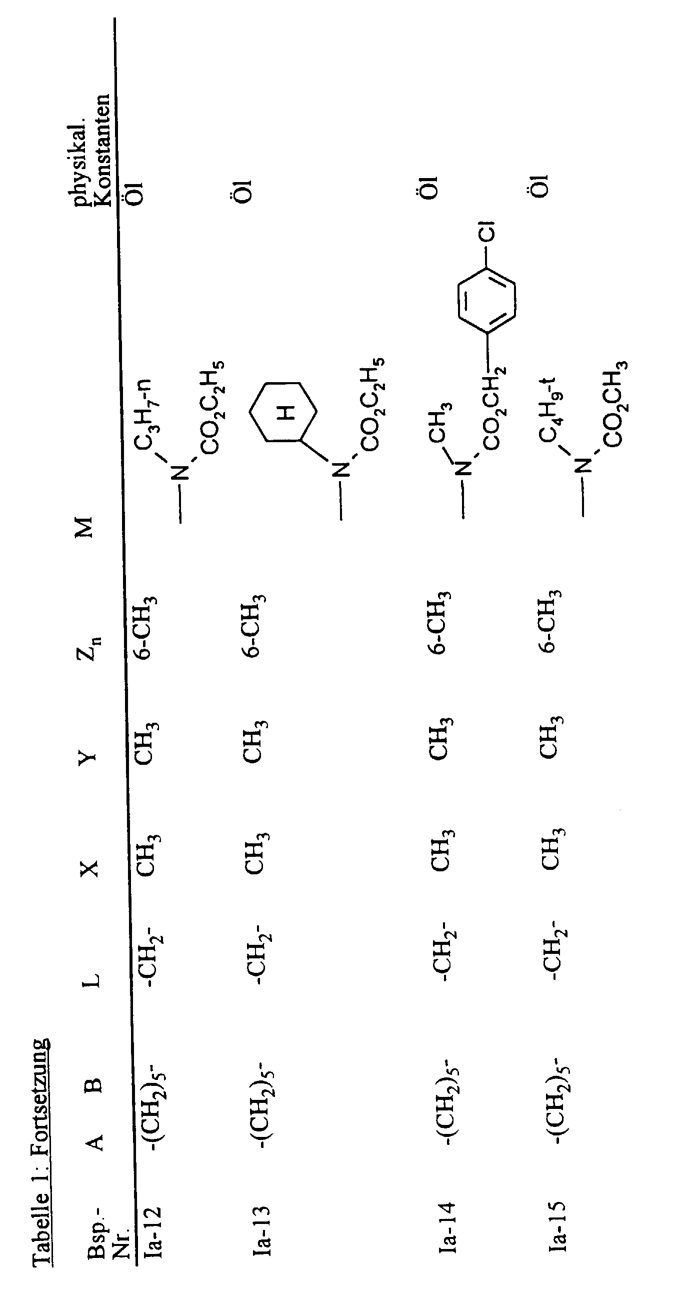

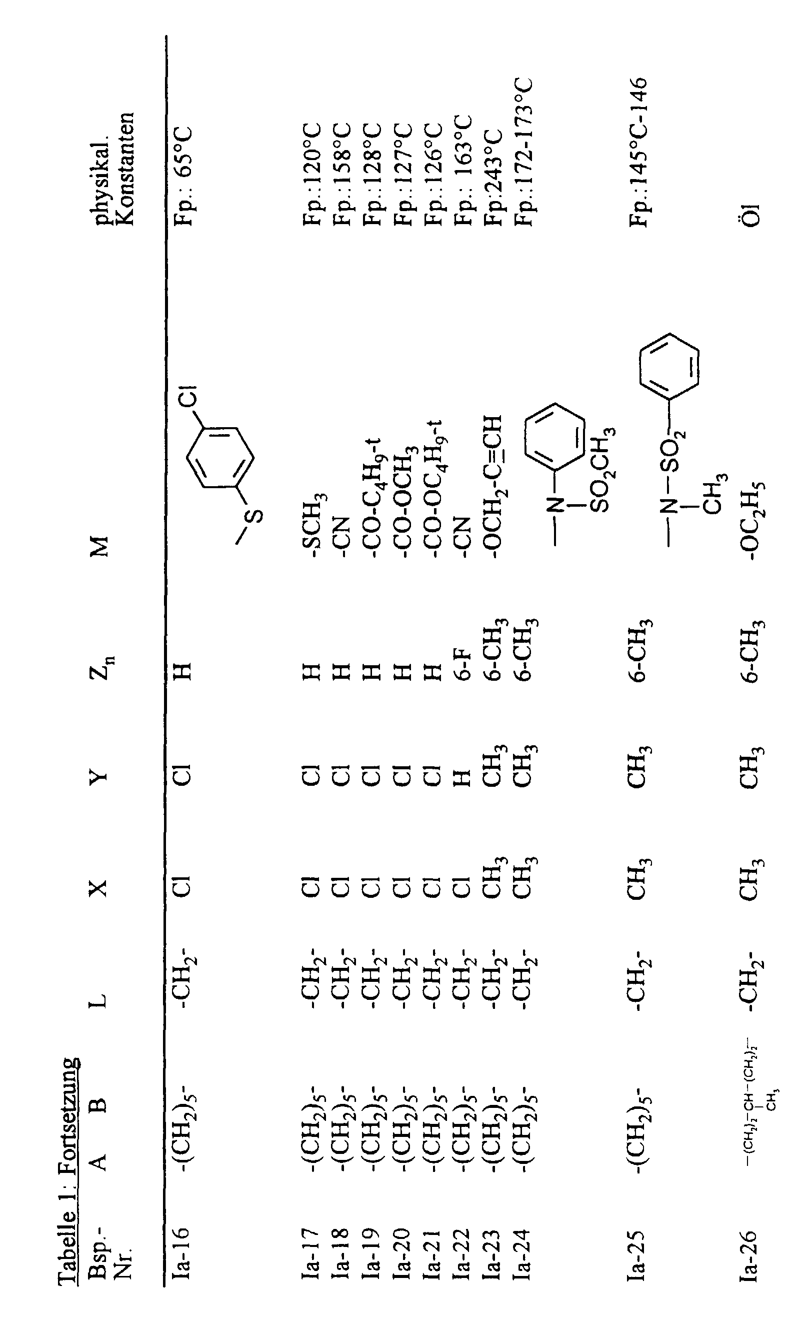

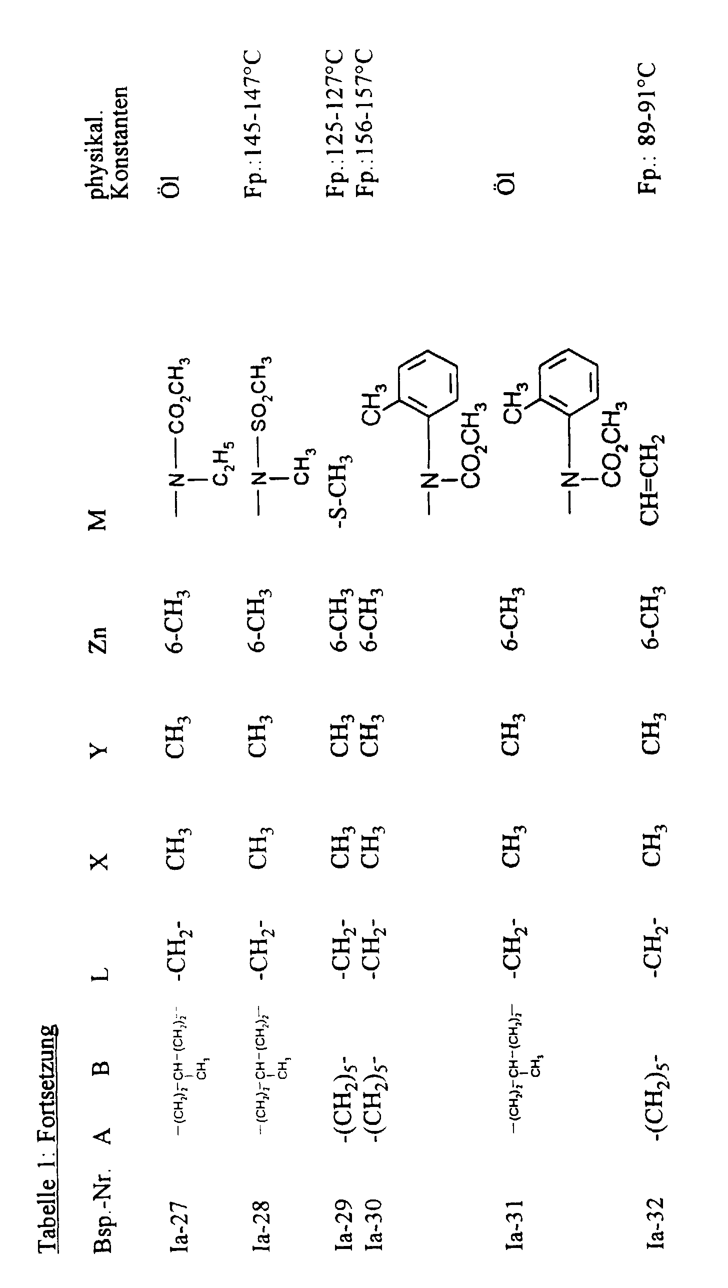

In Analogie zu Beispiel Ia-1 wird auch das Herstellungsbeispiel Ib-1 erhalten:

¹H-NMR (200 MHz, CDCl₃): 2.12, 2.2, 2.26 (3s, 9H, Ar-CH₃), 3.88 (s, 3H, SO₂, CH₃), 3.92 (s, 3H, N-CH₃), 3.25, 3.62 (m, 2H, N-CH₂), 4.21 (dd, 1H, CH-N), 4.72, (ABq, 2H, O-CH₂N), 6.89 (s, 2H, Ar-H).

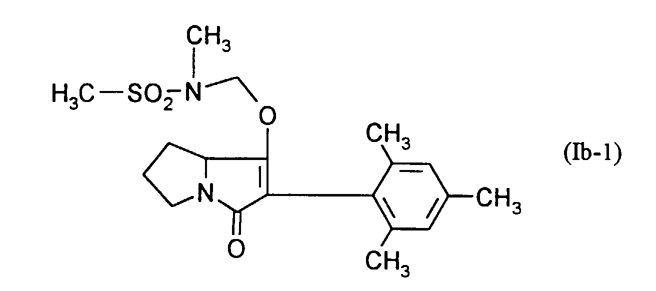

In Analogie zu Beispiel Ia-1 wird auch das Herstellungsbeispiel der Formel (Ic-1), Schmelzpunkt Fp. 207°C erhalten.

### Beispiel Ic-1

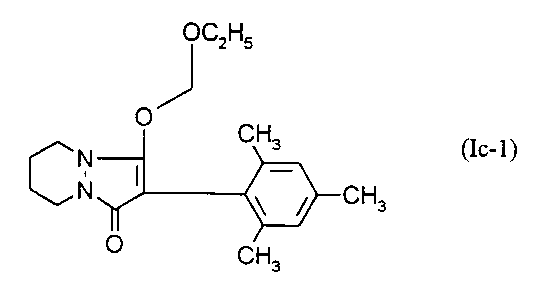

### Anwendungsbeispiele:

In den nachfolgenden Anwendungsbeispielen wurde die nachstehend aufgeführte Verbindung als Vergleichssubstanz eingesetzt: 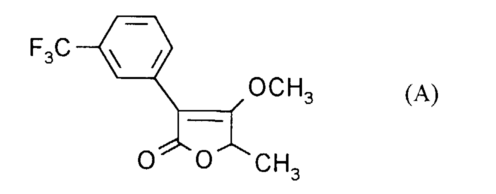 4-Methoxy-3-(3trifluormethylphenyl)-5-methyl-5H-furan-2-on (bekannt aus DE 40 14 420, Beispiel 6).

### Beispiel A:

| Panonychus-Test | |
|---|---|
| Lösungsmittel | 3 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschte Konzentration.

Ca. 30 cm hohe Pflaumenbäumchen (Prunus domestica), die stark von allen Entwicklungsstadien der Obstbaumspinnmilbe (Panonychus ulmi) befallen sind, werden mit einer Wirkstoffzubereitung der gewünschten Konzentration gespritzt.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

Bei diesem Test bewirkten z.B. die Verbindungen gemäß den Herstellungsbeispielen Ia-1, Ia-2, Ia-5 und Ia-7 bei einer beispielhaften Wirkstoffkonzentration von 0,02 % eine Abtötung von 100 % nach 7 Tagen.

### Beispiel B

| Tetranychus-Test (OP-resistent) | |
|---|---|
| Lösungsmittel | 3 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test bewirkten z.B. die Verbindungen gemäß den Herstellungsbeispielen Ia-1, Ia-2, Ia-5 und Ia-7 bei einer beispielhaften Wirkstoffkonzentration von 0,02 % eine Abtötung von mindestens 98 % nach 7 Tagen.

### Beispiel C

| Pre-emergence-Test | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigen beispielsweise die Verbindungen gemäß Herstellungsbeispiel (Ia-1) und (Ia-2) bei teilweise sehr guter Verträglichkeit gegenüber Kulturpflanzen, wie z.B. Weizen und Soja, sehr starke Wirkung gegen Unkräuter. Bei einer beispielhaften Aufwandmenge von 1000 g/ha werden z.B. Alopecurus, Cynodon, Digitaria, Lolium und Setaria zu mindestens 90 % erfaßt.

### Beispiel D

| Phaedon-Larven-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

In diesem Test bewirkten z.B. die Verbindungen gemäß den Herstellungsbeispielen Ia-9, Ia-12, Ia-13, Ia-14, Ia-15, Ib-1 und Ic-1 bei einer beispielhaften Wirkstoffkonzentration von 0,01 % eine Abtötung von 100 % nach 7 Tagen, während die bekannte Verbindung (A) keine Abtötung bewirkte.

### Beispiel E

| Plutella-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß den Herstellungsbeispielen Ia-14 und Ia-27 bei einer beispielhaften Wirkstoffkonzentration von 0,01 % eine Abtötung von 100 % nach 3 Tagen, während die bekannte Verbindung (A) keine Abtötung bewirkte.

### Beispiel F

| Nephotettix-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

In diesem Test bewirkten z.B. die Verbindungen gemäß den Herstellungsbeispielen Ia-1, Ia-3, Ia-9, Ia-12, Ia-13, Ia-15, Ia-25, Ia-26, Ia-27, Ia-28, Ia-31, Ib-1 und Ic-1 bei einer beispielhaften Wirkstoffkonzentration von 0,01 % eine Abtötung von 100 % nach 6 Tagen, während die bekannte Verbindung (A) keine Abtötung bewirkte.

### Beispiel G

| Myzus-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel- und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Wirkung in % bestimmt. Dabei bedeutet 100 %, daß alle Blattläuse abgetötet wurden; 0 % bedeutet, daß keine Blattläuse abgetötet wurden.

In diesem Test bewirkten z.B. die Verbindungen gemäß den Herstellungsbeispielen Ia-1, Ia-28 und Ia-31 bei einer beispielhaften Wirkstoffkonzentration von 0,1 % eine Abtötung von 100 % nach 6 Tagen, während die bekannte Verbindung (A) keine Abtötung bewirkte.

## Patentansprüche

1. Substituierte Aryl-ketoenolheterocyclen der Formel (I) 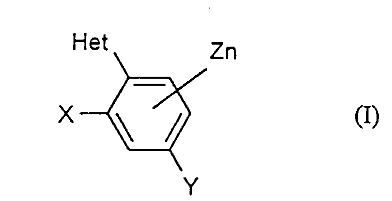 in welcher
X für Alkyl, Halogen oder Alkoxy steht,
Y für Wasserstoff, Alkyl, Halogen, Alkoxy oder Halogenalkyl steht,
Z für Alkyl, Halogen oder Alkoxy steht,
n für eine Zahl 0, 1, 2 oder 3 steht,
Het für eine heterocyclische Gruppe aus der Reihe 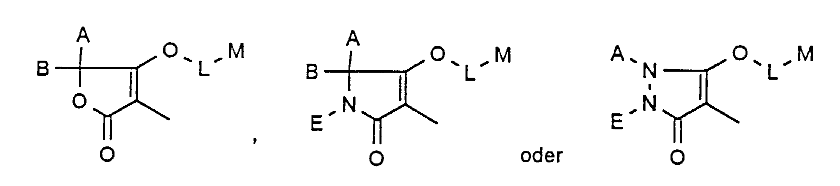
steht,
A und B gleich oder verschieden sein können und für Wasserstoff, jeweils gegebenenfalls durch Halogen substituiertes Alkyl, Alkenyl, Alkoxyalkyl, Alkylthioalkyl, gegebenenfalls durch mindestens ein Heteroatom unterbrochenes Cycloalkyl oder jeweils gegebenenfalls durch Halogen, Alkyl, Halogenalkyl, Alkoxy, Halogenalkoxy oder Nitro substituiertes Aryl, Aralkyl oder Hetaryl stehen,
oder worin
A und B gemeinsam mit dem Kohlenstoffatom, an das sie gebunden sind, einen gesättigten oder ungesättigten, gegebenenfalls durch mindestens ein Heteroatom unterbrochenen und gegebenenfalls substituierten Cyclus bilden,
E für Wasserstoff, jeweils gegebenenfalls durch Halogen substituiertes Alkyl, Alkenyl, Alkoxyalkyl, Alkylthioalkyl, gegebenenfalls durch mindestens ein Heteroatom unterbrochenes Cycloalkyl oder jeweils gegebenenfalls durch Halogen, Alkyl, Halogenalkyl, Alkoxy, Halogenalkoxy oder Nitro substituiertes Aryl, Aralkyl oder Hetaryl steht,
oder worin
A und E gemeinsam mit den Atomen, an die sie gebunden sind, einen gesättigten oder ungesättigten, gegebenenfalls durch mindestens ein Heteroatom unterbrochenen und gegebenenfalls substituierten Cyclus, Bicyclus oder Tricyclus bilden,
L für eine Alkandiylgruppe steht,
M für eine der folgenden Gruppierungen steht: 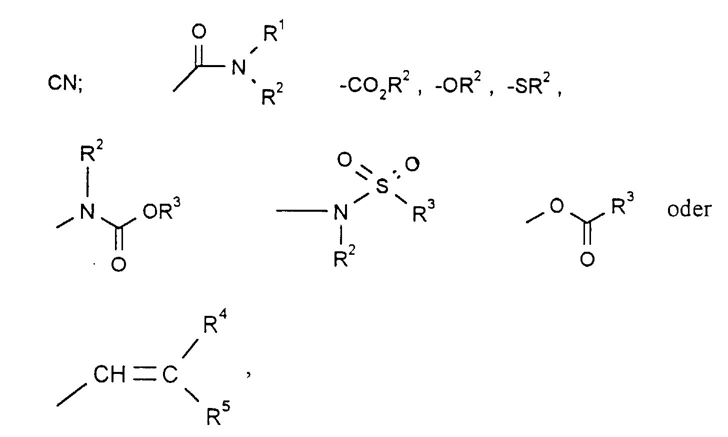
wobei
R¹ für Wasserstoff oder Alkyl steht,
R² für jeweils gegebenenfalls substituiertes Alkyl, Alkenyl, Alkinyl, Cycloalkyl, Alkoxyalkyl, Alkylthioalkyl, Aryl oder Aralkyl steht,
R³ für gegebenenfalls substituiertes Alkyl, Aryl oder Aralkyl steht,
R⁴ für Wasserstoff, Halogen, jeweils gegebenenfalls substituiertes Alkyl oder Phenyl steht und
R⁵ für Wasserstoff, Halogen oder gegebenenfalls substituiertes Alkyl steht,
sowie die enantiomerenreinen Formen von Verbindungen der Formel (I).

2. Verbindungen der Formel (I) gemäß Anspruch 1, in welcher
X für C₁-C₆-Alkyl, Halogen oder C₁-C₆-Alkoxy steht,
Y für Wasserstoff, C₁-C₆-Alkyl, Halogen, C₁-C₆-Alkoxy oder C₁-C₃-Halogenalkyl steht,
Z für C₁-C₆-Alkyl, Halogen oder C₁-C₆-Alkoxy steht,
n für eine Zahl von 0 bis 3 steht,
Het für eine heterocyclische Gruppe aus der Reihe 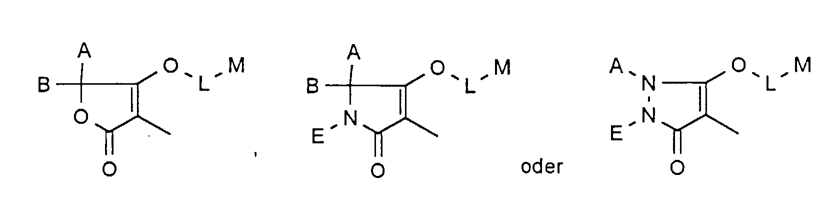
steht, worin
A und B gleich oder verschieden sind und für Wasserstoff oder jeweils gegebenenfalls durch Halogen substituiertes geradkettiges oder verzweigtes C₁-C₁₂-Alkyl, C₃-C₈-Alkenyl, C₁-C₁₀-Alkoxy-C₂-C₈-alkyl, C₁-C₈-Polyalkoxy-C₂-C₈-alkyl, C₁-C₁₀-Alkylthio-C₂-C₈-alkyl, Cycloalkyl mit 3 bis 8 Ringatomen, das durch Sauerstoff und/oder Schwefel unterbrochen sein kann oder jeweils gegebenenfalls durch Halogen, C₁-C₆-Alkyl, C₁-C₆-Haloalkyl, C₁-C₆-Alkoxy, C₁-C₆-Halogenalkoxy oder Nitro substituiertes Phenyl, Pyrimidyl, Imidazolyl, Pyrazolyl, Triazolyl, Indolyl, Thiazolyl oder Phenyl-C₁-C₆-alkyl steht,
oder worin
A und B gemeinsam mit dem Kohlenstoffatom, an das sie gebunden sind, einen gesättigten oder ungesättigten, gegebenenfalls durch Sauerstoff und/oder Schwefel unterbrochenen und gegebenenfalls durch Halogen, C₁-C₆-Alkyl, C₁-C₆-Alkoxy, C₁-C₄-Halogenalkyl, C₁-C₄-Halogenalkoxy, C₁-C₄-Alkylthio oder gegebenenfalls durch Halogen, C₁-C₆-Alkyl, C₁-C₆-Alkoxy substituiertes Phenyl substituierten 3- bis 8-gliedrigen Ring bilden,
E für Wasserstoff oder jeweils gegebenenfalls durch Halogen substituiertes geradkettiges oder verzweigtes C₁-C₁₂-Alkyl, C₃-C₈-Alkenyl, C₁-C₁₀-Alkoxy-C₂-C₈-alkyl, C₁-C₈-Polyalkoxy-C₂-C₈-alkyl, C₁-C₁₀-Alkylthio-C₂-C₈-alkyl, Cycloalkyl mit 3 bis 8 Ringatomen, das durch Sauerstoff und/oder Schwefel unterbrochen sein kann oder jeweils gegebenenfalls durch Halogen, C₁-C₆-Alkyl, C₁-C₆-Halogenalkyl, C₁-C₆-Alkoxy, C₁-C₆-Halogenalkoxy oder Nitro substituiertes Phenyl, Pyrimidyl, Imidazolyl, Pyrazolyl, Triazolyl, Indolyl, Thiazolyl oder Phenyl-C₁-C₆-alkyl steht,
oder worin
A und E gemeinsam mit den Atomen, an die sie gebunden sind, einen gesättigten oder ungesättigten, gegebenenfalls durch Sauerstoff und/ oder Schwefel unterbrochenen und gegebenenfalls durch Halogen, C₁-C₆-Alkyl, C₁-C₆-Alkoxy, C₁-C₄-Halogenalkyl, C₁-C₄-Halogenalkoxy, C₁-C₄-Alkylthio oder gegebenenfalls durch Halogen, C₁-C₆-Alkyl, C₁-C₆-Alkoxy substituiertes Phenyl substituierten 3- bis 8-gliedrigen Ring bilden,
oder für den Fall, daß Het in Formel (I) für einen Pyrazolinonring steht
A und E zusammen mit den beiden Stickstoffatomen des Pyrazolinrings für eine gegebenenfalls einfach oder mehrfach, gleich oder verschieden durch C₁-C₆-Alkyl, C₁-C₆-Halogenalkyl oder Halogen substituierte Gruppierung der nachfolgend aufgeführten Formeln 1 bis 4 stehen 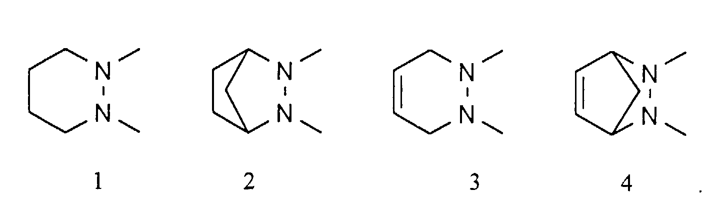
L für eine Alkandiylgruppe mit 1 bis 6 Kohlenstoffatomen steht,
M für eine der folgenden Gruppierungen steht: 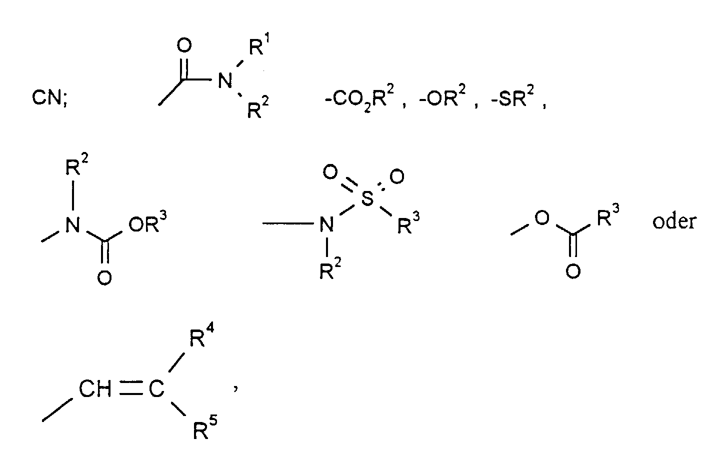
R¹ für Wasserstoff oder C₁-C₁₂-Alkyl steht,
R² für jeweils gegebenenfalls durch Halogen substituiertes C₁-C₁₂-Alkyl, C₃-C₈-Alkenyl, C₃-C₈-Alkinyl, C₃-C₈-Cycloalkyl, C₁-C₈-Alkoxy-C₂-C₈-alkyl, C₁-C₈-Alkylthio-C₂-C₈-alkyl, für jeweils gegebenenfalls durch Halogen, Nitro, Cyano, C₁-C₆-Alkoxy, C₁-C₆-Halogenalkoxy, C₁-C₆-Alkylthio, C₁-C₆-Halogenalkylthio, C₁-C₆-Alkyl oder C₁-C₆-Halogenalkyl substituiertes Phenyl oder Benzyl steht,
R³ für gegebenenfalls durch Halogen substituiertes C₁-C₁₂-Alkyl oder für jeweils durch Halogen, C₁-C₆-Alkyl oder C₁-C₆-Alkoxy substituiertes Phenyl oder Benzyl steht,
R⁴ für Wasserstoff, Halogen, gegebenenfalls durch Halogen substituiertes C₁-C₆-Alkyl oder jeweils durch Halogen, C₁-C₆-Alkyl, C₁-C₆-Alkoxyl, C₁-C₄-Haloalkyl, C₁-C₄-Haloalkoxy, Cyano oder Nitro substituiertes Phenyl steht und
R⁵ für Wasserstoff, Halogen oder gegebenenfalls durch Halogen substituiertes C₁-C₆-Alkyl steht,
sowie die enantiomerenreinen Formen von Verbindungen der Formel (I).

3. Verbindungen der Formel (I) gemäß Anspruch 1, in welcher
X für C₁-C₆-Alkyl, Fluor, Chlor, Brom oder C₁-C₆-Alkoxy steht,
Y für Wasserstoff, C₁-C₆-Alkyl, Fluor, Chlor, Brom, C₁-C₆-Alkoxy oder C₁-C₂-Halogenalkyl steht,
Z für C₁-C₄-Alkyl, Fluor, Chlor, Brom oder C₁-C₄-Alkoxy steht,
n für eine Zahl von 0 bis 2 steht,
Het für eine heterocyclische Gruppe der Reihe 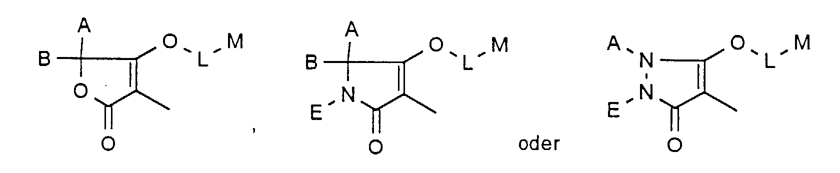
steht, worin
A und B gleich oder verschieden sind und für Wasserstoff, jeweils gegebenenfalls durch Halogen substituiertes geradkettiges oder verzweigtes C₁-C₁₀-Alkyl, C₃-C₆-Alkenyl, C₁-C₈-Alkoxy-C₂-C₆-alkyl, C₁-C₆-Polyalkoxy-C₂-C₆-alkyl, C₁-C₈-Alkylthio-C₂-C₆-alkyl, Cycloalkyl mit 3 bis 7 Ringatomen, das durch 1-2 Sauerstoff- und/oder Schwefelatome unterbrochen sein kann oder jeweils gegebenenfalls durch Halogen, C₁-C₄-Alkyl, C₁-C₄-Halogenalkyl, C₁-C₄-Alkoxy oder Nitro substituiertes Phenyl, Pyridinyl, Imidazolyl, Pyrazolyl, Triazolyl, Indolyl, Thiazolyl oder Phenyl-C₁-C₄-alkyl stehen,
oder worin
A und B gemeinsam mit dem Kohlenstoffatom, an das sie gebunden sind, einen gesättigten oder ungesättigten, gegebenenfalls durch Sauerstoff und/oder Schwefel unterbrochenen und gegebenenfalls durch Halogen, C₁-C₅-Alkyl, C₁-C₅-Alkoxy, C₁-C₃-Halogenalkyl, C₁-C₄-Halogenalkoxy, C₁-C₃-Alkylthio oder gegebenenfalls durch Halogen, C₁-C₄-Alkyl, C₁-C₄-Alkoxy substituiertes Phenyl substituierten 3- bis 8-gliedrigen Ring bilden,
E für Wasserstoff oder jeweils gegebenenfalls durch Halogen substituiertes geradkettiges oder verzweigtes C₁-C₁₀-Alkyl, C₃-C₆-Alkenyl, C₁-C₈-Alkoxy-C₂-C₆-alkyl, C₁-C₆-Polyalkoxy-C₂-C₆-alkyl, C₁-C₈-Alkylthio-C₂-C₆-alkyl, Cycloalkyl mit 3 bis 7 Ringatomen, das durch 1-2 Sauerstoff- und/oder Schwefelatome unterbrochen sein kann oder jeweils gegebenenfalls durch Halogen, C₁-C₄-Alkyl, C₁₋C₄-Halogenalkyl, C₁-C₄-Alkoxy oder Nitro substituiertes Phenyl, Pyridinyl, Imidazolyl, Pyrazolyl, Triazolyl, Indolyl, Thiazolyl oder Phenyl-C₁-C₄-alkyl steht,
oder worin
A und E gemeinsam mit den Atomen, an die sie gebunden sind, einen gesättigten oder ungesättigten, gegebenenfalls durch Sauerstoff und/oder Schwefel unterbrochenen und gegebenenfalls durch Halogen, C₁-C₅-Alkyl, C₁-C₅-Alkoxy, C₁-C₃-Halogenalkyl, C₁-C₄-Halogenalkoxy, C₁-C₃-Alkylthio oder gegebenenfalls durch Halogen, C₁-C₄-Alkyl, C₁-C₄-Alkoxy substituiertes Phenyl substituierten 3- bis 8-gliedrigen Ring bilden,
oder für den Fall, daß in Formel (I) Het für einen Pyrazolinonring steht
A und E zusammen mit den beiden Stickstoffatomen des Pyrazolinrings für eine gegebenenfalls einfach oder mehrfach, gleich oder verschieden durch C₁-C₄-Alkyl, C₁-C₄-Halogenalkyl, Fluor oder Chlor substituierte Gruppierung der nachfolgend aufgeführten Formeln 1 oder 2 stehen, 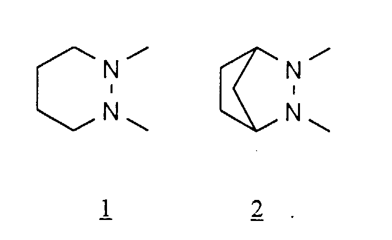
L für eine Alkandiylgruppe mit 1 bis 4 Kohlenstoffatomen steht,
M für eine der folgenden Gruppierungen steht: 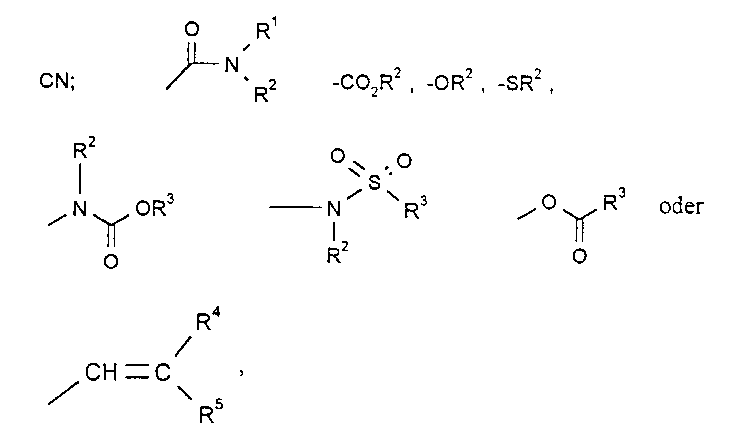
R¹ für Wasserstoff oder C₁-C₁₀-Alkyl steht,
R² für jeweils gegebenenfalls durch Fluor, Chlor, Brom substituiertes C₁-C₁₀-Alkyl, C₃-C₆-Alkenyl, C₃-C₆-Alkinyl, C₃-C₈-Cycloalkyl, C₁-C₆-Alkoxy-C₂-C₆-alkyl, C₁-C₆-Alkylthio-C₂-C₆-alkyl, für jeweils gegebenenfalls durch Fluor, Chlor, Brom, Nitro, Cyano, C₁-C₄-Alkylthio, C₁-C₄-Alkoxy, C₁-C₄-Halogenalkoxy, C₁-C₄-Halogenalkylthio, C₁-C₄-Alkyl oder C₁-C₄-Halogenalkyl substituiertes Phenyl oder Benzyl steht,
R³ für gegebenenfalls durch Fluor, Chlor, Brom substituiertes C₁-C₁₀-Alkyl oder für jeweils durch Fluor, Chlor, C₁-C₄-Alkyl oder C₁-C₄-Alkoxy substituiertes Phenyl oder Benzyl steht,
R⁴ für Wasserstoff, Fluor, Chlor, Brom, gegebenenfalls durch Fluor oder Chlor substituiertes C₁-C₅-Alkyl oder durch Fluor, Chlor, Brom, C₁-C₄-Alkyl, C₁-C₄-Alkoxy, C₁-C₂-Haloalkyl, C₁-C₂-Haloalkoxy, Cyano oder Nitro substituiertes Phenyl steht und
R⁵ für Wasserstoff, Fluor, Chlor, Brom oder gegebenenfalls durch Fluor oder Chlor substituiertes C₁-C₄-Alkyl steht,
sowie die enantiomerenreinen Formen von Verbindungen der Formel (I).

4. Verbindungen der Formel (I) gemäß Anspruch 1, in welcher
X für Methyl, Ethyl, Propyl, i-Propyl, Fluor, Chlor, Brom oder Methoxy steht,
Y für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,
Z für Methyl, Ethyl, Propyl, i-Propyl, Butyl, i-Butyl, tert.-Butyl, Fluor, Chlor, Brom, Methoxy und Ethoxy steht,
n für 1 steht,
Het für eine heterocyclische Gruppe aus der Reihe 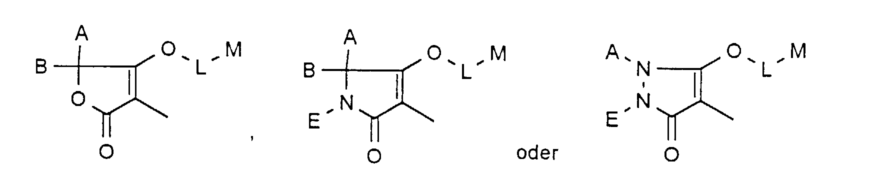
steht, worin
A und B gleich oder verschieden sind und für Wasserstoff, jeweils gegebenenfalls durch Fluor oder Chlor substituiertes geradkettiges oder verzweigtes C₁-C₈-Alkyl, C₃-C₄-Alkenyl, C₁-C₆-Alkoxy-C₂-C₂-alkyl, C₁-C₄-Polyalkoxy-C₂-C₄-alkyl, C₁-C₆-Alkylthio-C₂-C₄-alkyl, Cycloalkyl mit 3 bis 6 Ringatomen, das durch 1-2 Sauerstoff- und/oder Schwefelatome unterbrochen sein kann oder jeweils gegebenenfalls durch Fluor, Chlor, Methyl, Ethyl, Propyl, iso-Propyl, Methoxy, Ethoxy, Trifluormethyl oder Nitro-substituiertes Phenyl, Pyridinyl, Imidazolyl, Indolyl oder Phenyl-C₁-C₃-alkyl stehen,
oder worin
A und B gemeinsam mit dem Kohlenstoffatom, an das sie gebunden sind, einen gesättigten oder ungesättigten, gegebenenfalls durch Sauerstoff und/oder Schwefel unterbrochenen und gegebenenfalls durch Fluor, Chlor, C₁-C₄-Alkyl, C₁-C₄-Alkoxy, Trifluormethyl, C₁-C₂-Alkylthio oder gegebenenfalls durch Fluor, Chlor, Methyl, Methoxy substituiertes Phenyl substituierten 3- bis 8-gliedrigen Ring bilden,
E für Wasserstoff oder jeweils gegebenenfalls durch Fluor oder Chlor substituiertes geradkettiges oder verzweigtes C₁-C₈-Alkyl, C₃-C₄-Alkenyl, C₁-C₆-Alkoxy-C₂-C₄-alkyl, C₁-C₄-Polyalkoxy-C₂-C₄-alkyl, C₁-C₆-Alkylthio-C₂-C₄-alkyl, Cycloalkyl mit 3 bis 6 Ringatomen, das durch 1-2 Sauerstoff- und/oder Schwefelatome unterbrochen sein kann oder gegebenenfalls durch Fluor, Chlor, Methyl, Ethyl, Propyl, iso-Propyl, Methoxy, Ethoxy, Trifluormethyl oder Nitro substituiertes Phenyl, Pyridinyl, Imidazolyl, Indolyl oder Phenyl-C₁-C₃-alkyl steht,
oder worin
A und E gemeinsam mit den Atomen, an die sie gebunden sind, einen gesättigten oder ungesättigten, gegebenenfalls durch Sauerstoff und/ oder Schwefel unterbrochenen und gegebenenfalls durch Fluor, Chlor, C₁-C₄-Alkyl, C₁-C₄-Alkoxy, Trifluormethyl, C₁-C₂-Alkylthio oder gegebenenfalls durch Fluor, Chlor, Methyl, Methoxy substituiertes Phenyl substituierten 3- bis 8-gliedrigen Ring bilden,
oder für den Fall, daß in Formel (I) Het für einen Pyrazolinonring steht
A und E zusammen mit den beiden Stickstoffatomen des Pyrazolinrings für eine gegebenenfalls einfach bis dreifach, gleich oder verschieden durch Methyl, Fluor oder Chlor substituierte Gruppierung der nachfolgend aufgeführten Formeln 1 oder 2 stehen, 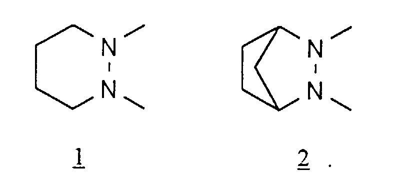
L für eine der folgenden Gruppierungen steht 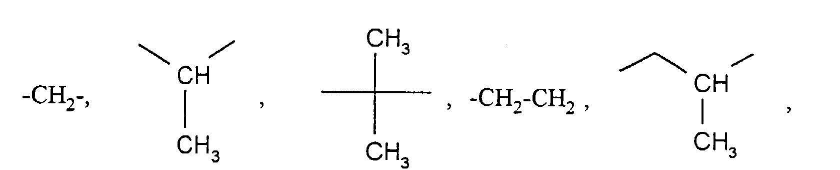
M für eine der folgenden Gruppierungen steht 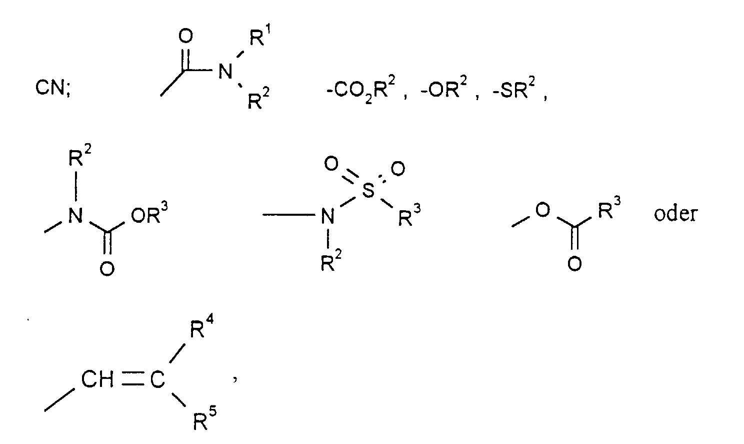
R¹ für Wasserstoff oder C₁-C₈-Alkyl steht,
R² für jeweils gegebenenfalls durch Fluor, Chlor, Brom substituiertes C₁-C₈-Alkyl, C₃-C₄-Alkenyl, C₃-C₄-Alkinyl, Cyclopropyl, Cyclopentyl, Cyclohexyl, C₁-C₆-Alkoxy-C₂-C₄-alkyl, C₁-C₆-Alkylthio-C₂-C₄-alkyl, für jeweils gegebenenfalls durch Fluor, Chlor, Brom, Nitro, Cyano, Methylthio, Ethylthio, Methoxy, Ethoxy, Trifluormethylthio, Trifluormethoxy, Methyl, Ethyl, Trifluormethyl substituiertes Phenyl oder Benzyl steht,
R³ für jeweils gegebenenfalls durch Fluor, Chlor, Brom substituiertes C₁-C₈-Alkyl oder durch Fluor, Chlor, C₁-C₂-Alkyl oder C₁-C₂-Alkoxy substituiertes Phenyl oder Benzyl steht,
R⁴ für Wasserstoff, Fluor, Chlor, gegebenenfalls durch Fluor oder Chlor substituiertes C₁-C₄-Alkyl oder durch Fluor, Chlor, Methyl, Ethyl, Methoxy, Ethoxy, Trifluormethyl, Difluormethoxy, Trifluormethoxy, Cyano oder Nitro substituiertes Phenyl steht und
R⁵ für Wasserstoff, Fluor, Chlor oder gegebenenfalls durch Fluor oder Chlor substituiertes Methyl, Ethyl, Propyl oder Isopropyl steht,
sowie die enantiomerenreinen Formen von Verbindungen der Formel (I).

5. Verfahren zur Herstellung der substituierten Aryl-ketoenolheterocyclen der Formel (I) gemäß Anspruch 1, dadurch gekennzeichnet, daß man (A) zum Erhalt von Verbindungen der Formel (Ia) 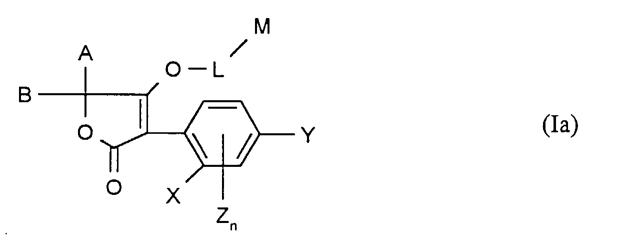 in welcher
A, B, L, M, X, Y, Z und n die in Anspruch 1 angegebene Bedeutung haben,
Verbindungen der Formel (IIa) 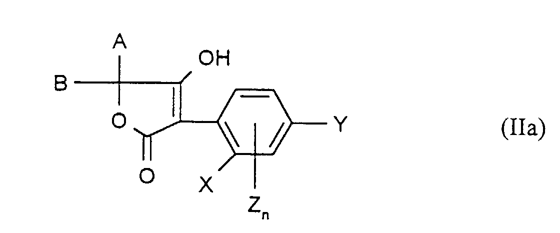 in welcher
A, B, X, Y, Z und n die oben angegebene Bedeutung haben,
mit Verbindungen der Formel (III)
G-L-M (III)
in welcher
L und M die oben angegebene Bedeutung haben
und
G für eine Abgangsgruppe steht,
in Gegenwart eines Verdünnungsmittels und in Gegenwart einer Base umsetzt,
(B) zum Erhalt von Verbindungen der Formel (Ib) 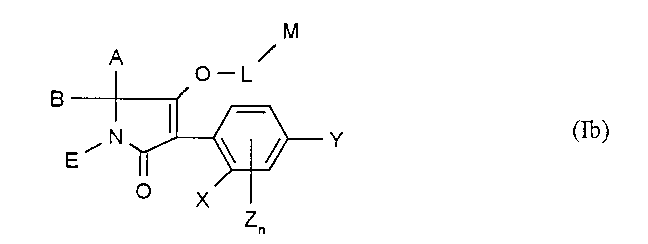 in welcher
A, B, E, L, M, X, Y, Z und n die oben angegebene Bedeutung haben,
Verbindungen der Formel (IIb) 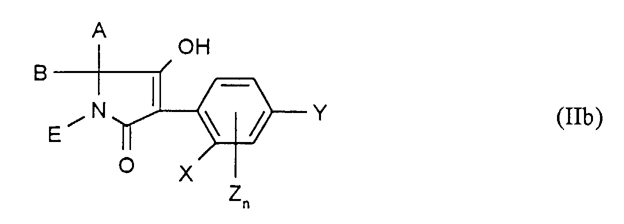 in welcher
A, B, E, X, Y, Z und n die oben angegebene Bedeutung haben,
mit Verbindungen der Formel (III)
G-L-M (III)
in welcher
L und M die oben angegebene Bedeutung haben
und
G für eine Abgangsgruppe steht,
in Gegenwart eines Verdünnungsmittels und in Gegenwart einer Base umsetzt oder
(C) zum Erhalt von Verbindungen der Formel (Ic) 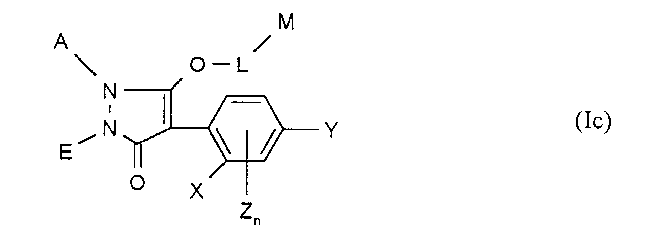 in welcher
A, E, L, M, X, Y, Z und n die oben angegebene Bedeutung haben,
Verbindungen der Formel (IIc) 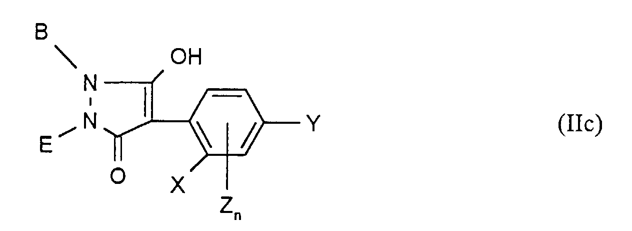 in welcher
B, E, X, Y, Z und n die oben angegebene Bedeutung haben,
mit Verbindungen der Formel (III)
G-L-M (III)
in welcher
L und M die oben angegebene Bedeutung haben
und
G für eine Abgangsgruppe steht,
in Gegenwart eines Verdünnungsmittels und in Gegenwart einer Base umsetzt.

6. Schädlingsbekämpfungsmittel, gekennzeichnet durch einen Gehalt an mindestens einer Verbindung der Formel (I) gemäß Anspruch 1.

7. Arthropodizide, fungizide und herbizide Mittel, gekennzeichnet durch einen Gehalt an mindestens einer Verbindung der Formel (I) gemäß Anspruch 1.

8. Verwendung von Verbindungen der Formel (I) gemäß Anspruch 1, zur Bekämpfung von Schädlingen im Pflanzenschutz, Haushaltsbereich, Hygienebereich und Vorratsschutz.

9. Verwendung von Verbindungen der Formel (I) gemäß Anspruch 1, zur Bekämpfung von Arthropoden, phytopatogenen Pilzen und unerwünschtem Pflanzenbewuchs im Pflanzenschutz, Haushaltsbereich, Hygienebereich und Vorratsschutz.

10. Verfahren zur Bekämpfung von Schädlingen im Pflanzenschutz, Haushaltsbereich, Hygienebereich und Vorratsschutz, dadurch gekennzeichnet, daß man Verbindungen der Formel (I) gemäß Anspruch 1 auf die Schädlinge und/oder ihren Lebensraum einwirken läßt.

11. Verfahren zur Herstellung von Schädlingsbekämpfungsmitteln, dadurch gekennzeichnet, daß man Verbindungen der Formel (I) gemäß Anspruch 1, mit Streckmitteln und/oder oberflächenaktiven Mitteln vermischt.

## Claims

1. Substituted aryl keto-enolic heterocycles of the formula (I) 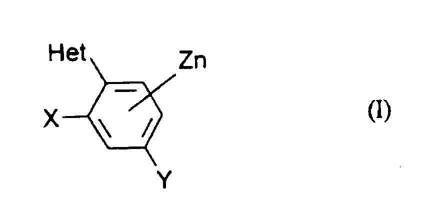 in which
X represents alkyl, halogen or alkoxy,
Y represents hydrogen, alkyl, halogen, alkoxy or halogenoalkyl,
Z represents alkyl, halogen or alkoxy,
n represents a number 0, 1, 2 or 3,
Het represents a heterocyclic group from the series 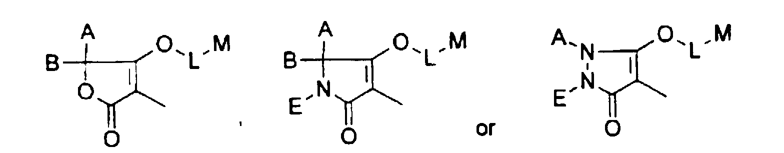
A and B can be identical or different and represent hydrogen, in each case optionally halogen-substituted alkyl, alkenyl, alkoxyalkyl, alkylthioalkyl, cycloalkyl which is optionally interrupted by at least one heteroatom, or aryl, aralkyl or hetaryl each of which is optionally substituted by halogen, alkyl, halogenoalkyl, alkoxy, halogenoalkoxy or nitro,
or in which
A and B, together with the carbon atom to which they are attached, form a saturated or unsaturated ring which is optionally interrupted by at least one heteroatom and is optionally substituted,
E represents hydrogen, in each case optionally halogen-substituted alkyl, alkenyl, alkoxyalkyl, alkylthioalkyl, cycloalkyl which is optionally interrupted by at least one heteroatom, or aryl, aralkyl or hetaryl each of which is optionally substituted by halogen, alkyl, halogenoalkyl, alkoxy, halogenoalkoxy or nitro,
or in which
A and E, together with the atoms to which they are attached, form a saturated or unsaturated monocyclic, bicyclic or tricyclic ring system which is optionally interrupted by at least one heteroatom and is optionally substituted,
L represents an alkanediyl group,
M represents one of the following groups: 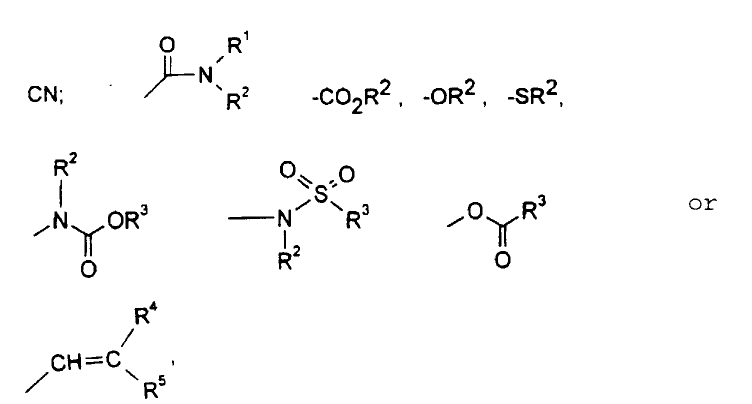
in which
R¹ represents hydrogen or alkyl,
R² represents in each case optionally substituted alkyl, alkenyl, alkinyl, cycloalkyl, alkoxyalkyl, alkylthioalkyl, aryl or aralkyl,
R³ represents optionally substituted alkyl, aryl or aralkyl,
R⁴ represents hydrogen, halogen, in each case optionally substituted alkyl or phenyl, and
R⁵ represents hydrogen, halogen or optionally substituted alkyl,
and the enantiomerically pure forms of compounds of the formula (I).

2. Compounds of the formula (I) according to Claim 1, in which
X represents C₁-C₆-alkyl, halogen or C₁-C₆-alkoxy,
Y represents hydrogen, C₁-C₆-alkyl, halogen C₁-C₆-alkoxy or C₁-C₃-halogenoalkyl,
Z represents C₁-C₆-alkyl, halogen or C₁-C₆-alkoxy,
n represents a number from 0 to 3,
Het represents a heterocyclic group from the series 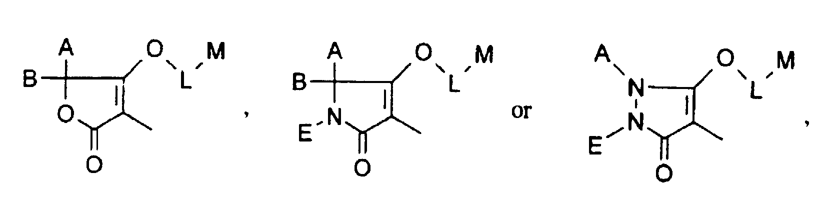
in which
A and B are identical or different and represent hydrogen or in each case optionally halogen-substituted straight-chain or branched C₁-C₁₂-alkyl, C₃-C₈-alkenyl, C₁-C₁₀-alkoxy-C₂-C₈-alkyl, C₁-C₈-polyalkoxy-C₂-C₈-alkyl, C₁-C₁₀-alkylthio-C₂-C₈-alkyl, cycloalkyl having 3 to 8 ring atoms, which can be interrupted by oxygen and/or sulphur, or phenyl, pyrimidyl, imidazolyl, pyrazolyl, triazolyl, indolyl, thiazolyl or phenyl-C₁-C₆-alkyl each of which is optionally substituted by halogen, C₁-C₆-alkyl, C₁-C₆-haloalkyl, C₁-C₆-alkoxy, C₁-C₆-halogenoalkoxy or nitro,
or in which
A and B, together with the carbon atom to which they are attached, form a saturated or unsaturated 3- to 8-membered ring which is optionally interrupted by oxygen and/or sulphur and is optionally substituted by halogen, C₁-C₆-alkyl, C₁-C₆ alkoxy, C₁-C₄-halogenoalkyl, C₁-C₄-halogenoalkoxy, C₁-C₄-alkylthio or phenyl which is optionally substituted by halogen, C₁-C₆-alkyl, C₁-C₆-alkoxy,
E represents hydrogen or in each case optionally halogen-substituted straight-chain or branched C₁-C₁₂-alkyl, C₃-C₈-alkenyl, C₁-C₁₀-alkoxy-C₂-C₈-alkyl, C₁-C₈-polyalkoxy-C₂-C₈-alkyl, C₁-C₁₀-alkylthio-C₂-C₈-alkyl, cycloalkyl having 3 to 8 ring atoms, which can be interrupted by oxygen and/or sulphur, or phenyl, pyrimidyl, imidazolyl, pyrazolyl, triazolyl, indolyl, thiazolyl or phenyl-C₁-C₆-alkyl each of which is optionally substituted by halogen, C₁-C₆-alkyl, C₁-C₆-haloalkyl, C₁-C₆-alkoxy, C₁-C₆-halogenoalkoxy or nitro,
or in which
A and E, together with the atoms to which they are attached, form a saturated or unsaturated 3- to 8-membered ring which is optionally interrupted by oxygen and/or sulphur and is optionally substituted by halogen, C₁-C₆-alkyl, C₁-C₆ alkoxy, C₁-C₄-halogenoalkyl, C₁-C₄-halogenoalkoxy, C₁-C₄-alkylthio or phenyl which is optionally substituted by halogen, C₁-C₆-alkyl, C₁-C₆-alkoxy,
or, if Het in formula (I) represents a pyrazolinone ring,
A and E, together with two nitrogen atoms of the pyrazoline ring, represent a group of the formulae 1 to 4 listed below 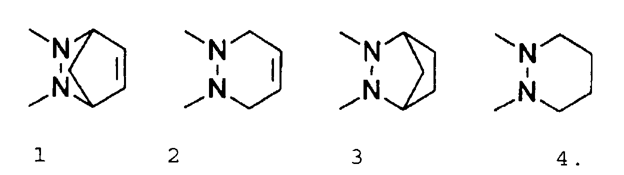 which is optionally monosubstituted or polysubstituted by identical or different substituents consisting of C₁-C₆-alkyl, C₁-C₆-halogenoalkyl or halogen,
L represents an alkanediyl group having 1 to 6 carbon atoms,
M represents one of the following: 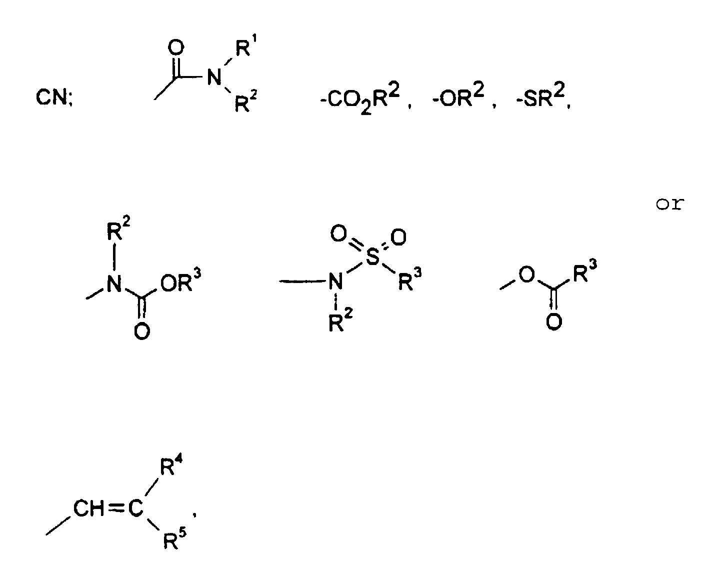
R¹ represents hydrogen or C₁-C₁₂-alkyl,
R² represents in each case optionally halogen-substituted C₁-C₁₂-alkyl, C₃-C₈-alkenyl, C₃-C₈-alkinyl, C₃-C₈-cycloalkyl, C₁-C₈-alkoxy-C₂-C₈-alkyl, C₁-C₈-alkylthio-C₂-C₄-alkyl or phenyl or benzyl each of which is optionally substituted by halogen, nitro, cyano, C₁-C₆-alkoxy, C₁-C₆-haloakylthio, C₁-C₆-alkyl or C₁-C₆-halogenoalkyl,
R³ represents optionally halogen-substituted C₁-C₁₂-alkyl, or represents phenyl or benzyl each of which is substituted by halogen, C₁-C₆-alkyl or C₁-C₆-alkoxy,
R⁴ represents hydrogen, halogen, optionally halogen-substituted C₁-C₆-alkyl or phenyl which is in each case substituted by halogen, C₁-C₆-alkyl or C₁-C₈-alkoxy, C₁-C₄-haloalkyl, C₁-C₄-haloalkoxy, cyano or nitro, and
R⁵ represents hydrogen, halogen or optionally halogen-substituted C₁-C₆-alkyl,
and the enantiomerically pure forms of the compound (I).

3. Compounds of the formula (I) according to Claim 1, in which
X represents C₁-C₆-alkyl, fluorine, chlorine, bromine or C₁-C₆-alkoxy,
Y represents hydrogen, C₁-C₆-alkyl, fluorine, chlorine, bromine, C₁-C₆-alkoxy or C₁-C₂-halogenoalkyl,
Z represents C₁-C₄-alkyl, fluorine, chlorine, bromine or C₁-C₄-alkoxy,
n represents a number from 0 to 2,
Het represents a heterocyclic group from the series 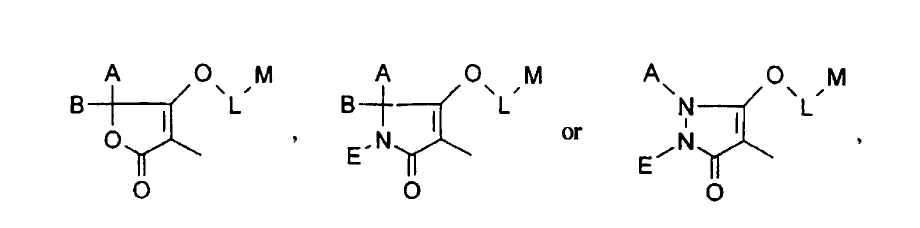
in which
A and B are identical or different and represent hydrogen, in each case optionally halogen-substituted straight-chain or branched C₁-C₁₀-alkyl, C₃-C₆-alkenyl, C₁-C₈-alkoxy-C₂-C₆-alkyl, C₁-C₆-polyalkoxy-C₂-C₆-alkyl, C₁-C₈-alkylthio-C₂-C₆-alkyl, cycloalkyl having 3 to 7 ring atoms, which can be interrupted by 1-2 oxygen and/or sulphur atoms, or phenyl, pyridinyl, imidazolyl, pyrazolyl, triazolyl, indolyl, thiazolyl or phenyl-C₁-C₄-alkyl each of which is optionally substituted by halogen, C₁-C₄-alkyl, C₁-C₄-haloalkyl, C₁-C₄-alkoxy, or nitro,
or in which
A and B, together with the carbon atom to which they are attached, form a saturated or unsaturated 3- to 8-membered ring which is optionally interrupted by oxygen and/or sulphur and is optionally substituted by halogen, C₁-C₅-alkyl, C₁-C₅ alkoxy, C₁-C₃-halogenoalkyl, C₁-C₄-halogenoalkoxy, C₁-C₃-alkylthio or phenyl which is optionally substituted by halogen, C₁-C₄-alkyl, C₁-C₄-alkoxy,
E represents hydrogen or in each case optionally halogen-substituted straight-chain or branched C₁-C₁₀-alkyl, C₃-C₆-alkenyl, C₁-C₈-alkoxy-C₂-C₆-alkyl, C₁-C₆-polyalkoxy-C₂-C₆-alkyl, C₁-C₈-alkylthio-C₂-C₆-alkyl, cycloalkyl having 3 to 7 ring atoms, which can be interrupted by 1-2 oxygen and/or sulphur atoms, or phenyl, pyridinyl, imidazolyl, pyrazolyl, triazolyl, indolyl, thiazolyl or phenyl-C₁-C₄-alkyl each of which is optionally substituted by halogen, C₁-C₄-alkyl, C₁₋C₄-halogenoalkyl, C₁-C₄-alkoxy or nitro,
or in which
A and E, together with the atoms to which they are attached, form a saturated or unsaturated 3- to 8-membered ring which is optionally interrupted by oxygen and/or sulphur and is optionally substituted by halogen, C₁-C₅-alkyl, C₁-C₅-alkoxy, C₁-C₃-halogenoalkyl, C₁-C₄-halogenoalkoxy, C₁-C₃-alkylthio or phenyl which is optionally substituted by halogen, C₁-C₄-alkyl, C₁-C₄-alkoxy,
or, if Het in formula (I) represents a pyrazolinone ring,
A and E, together with the two nitrogen atoms of the pyrazoline ring, represent a group of the formulae 1 or 2 listed below which is optionally monosubstituted or polysubstituted by identical or different substituents consisting of C₁-C₄-alkyl, C₁-C₄-halogenoalkyl, fluorine or chlorine, 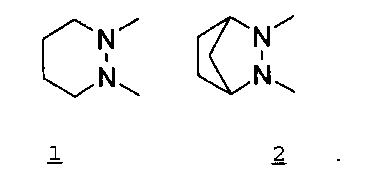
L represents an alkanediyl group having 1 to 4 carbon atoms,
M represents one of the following groups: 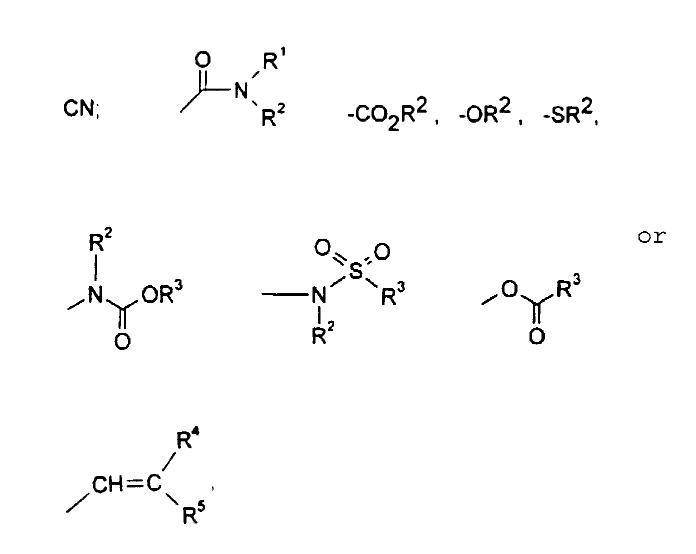
R¹ represents hydrogen or C₁-C₁₀-alkyl,
R² represents in each case optionally fluorine-, chlorine-, bromine-substituted C₁-C₁₀-alkyl, C₃-C₆-alkenyl, C₃-C₆-alkinyl, C₃-C₈-cycloalkyl, C₁-C₆-alkoxy-C₂-C₆-alkyl, C₁-C₆-alkylthio-C₂-C₆-alkyl, or phenyl or benzyl each of which is optionally substituted by fluorine, chlorine, bromine, nitro, cyano, C₁-C₄-alkylthio, C₁-C₄-alkoxy, C₁-C₄-halogenoalkoxy, C₁-C₄-halogenoalkylthio, C₁-C₄-alkyl or C₁-C₄-halogenoalkyl,
R³ represents optionally fluorine-, chlorine-, bromine-substituted C₁-C₁₀-alkyl, or represents phenyl or benzyl each of which is substituted by fluorine, chlorine, C₁-C₄-alkyl or C₁-C₄-alkoxy,
R⁴ represents hydrogen, fluorine, chlorine, bromine, optionally fluorine- or chlorine-substituted C₁-C₅-alkyl or phenyl which is substituted by fluorine, chlorine, bromine, C₁-C₄-alkyl or C₁-C₄-alkoxy, C₁-C₂-haloalkyl, C₁-C₂-haloalkoxy, cyano or nitro, and
R⁵ represents hydrogen, fluorine, chlorine, bromine or optionally fluorine- or chlorine-substituted C₁-C₄-alkyl,
and the enantiomerically pure form of the compounds of the formula (I).

4. Compounds of the formula (I) according to Claim 1, in which
X represents methyl, ethyl, propyl, isopropyl, fluorine, chlorine, bromine or methoxy,
Y represents hydrogen, methyl, ethyl, propyl, isopropyl, butyl, isobutyl, tert-butyl, fluorine, chlorine, bromine, methoxy, ethoxy and trifluoromethyl,
Z represents methyl, ethyl, propyl, isopropyl, butyl, isobutyl, tert-butyl, fluorine, chlorine, bromine, methoxy and ethoxy,
n represents 1,
Het represents a heterocyclic group from the series 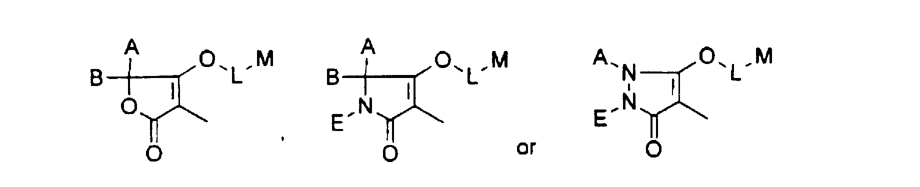
in which
A and B are identical or different and represent hydrogen, in each case optionally fluorine- or chlorine-substituted straight-chain or branched C₁-C₈-alkyl, C₃-C₄-alkenyl, C₁-C₆-alkoxy-C₂-C₄-alkyl, C₁-C₄-polyalkoxy-C₂-C₄-alkyl, C₁-C₆-alkylthio-C₂-C₄-alkyl, cycloalkyl having 3 to 6 ring atoms, which can be interrupted by 1-2 oxygen and/or sulphur atoms, or phenyl, pyridinyl, imidazolyl, indolyl or phenyl-C₁-C₃-alkyl each of which is optionally substituted by fluorine, chlorine, methyl, ethyl, propyl, isopropyl, methoxy, ethoxy, trifluoromethyl or nitro,
or in which
A and B, together with the carbon atom to which they are attached, form a saturated or unsaturated 3- to 8-membered ring which is optionally interrupted by oxygen and/or sulphur and is optionally substituted by fluorine, chlorine, C₁-C₄-alkyl, C₁-C₄ alkoxy, trifluoromethyl, C₁-C₂-alkylthio or phenyl which is optionally substituted by fluorine, chlorine, methyl, methoxy,
E represents hydrogen or in each case optionally fluorine- or chlorine-substituted straight-chain or branched C₁-C₈-alkyl, C₃-C₄-alkenyl, C₁-C₆-alkoxy-C₂-C₄-alkyl, C₁-C₄-polyalkoxy-C₂-C₄-alkyl, C₁-C₆-alkylthio-C₂-C₄-alkyl, cycloalkyl having 3 to 6 ring atoms, which can be interrupted by 1-2 oxygen and/or sulphur atoms, or represents phenyl, pyridinyl, imidazolyl, indolyl or phenyl-C₁-C₄-alkyl which is optionally substituted by fluorine, chlorine, methyl, ethyl, propyl, isopropyl, methoxy, ethoxy, trifluoromethyl or nitro,
or in which
A and E, together with the atoms to which they are attached, form a saturated or unsaturated 3- to 8-membered ring which is optionally interrupted by oxygen and/or sulphur and is optionally substituted by fluorine, chlorine, C₁-C₄-alkyl, C₁-C₄-alkoxy, trifluoromethyl, C₁-C₂-alkylthio or phenyl which is optionally substituted by fluorine, chlorine, methyl, methoxy,
or, if Het in formula (I) represents a pyrazolinone ring,
A and E, together with the two nitrogen atoms of the pyrazoline ring, represent a group of the formulae 1 or 2 listed below which is optionally monosubstituted to trisubstituted by identical or different substituents consisting of methyl, fluorine or chlorine, 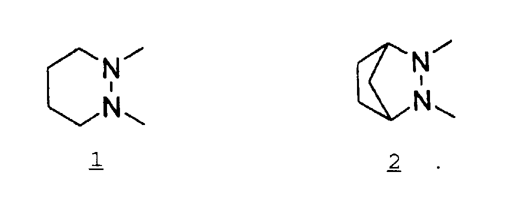
L represents one of the following groups, 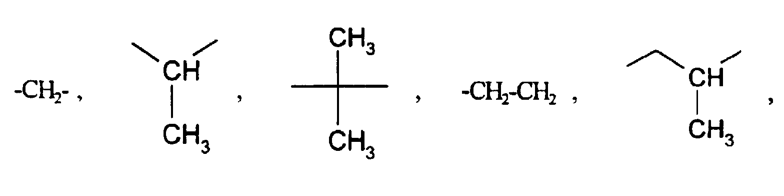
M represents one of the following groups: 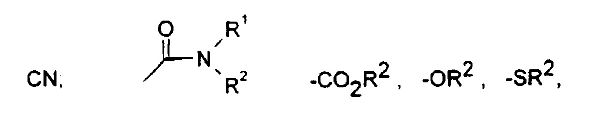 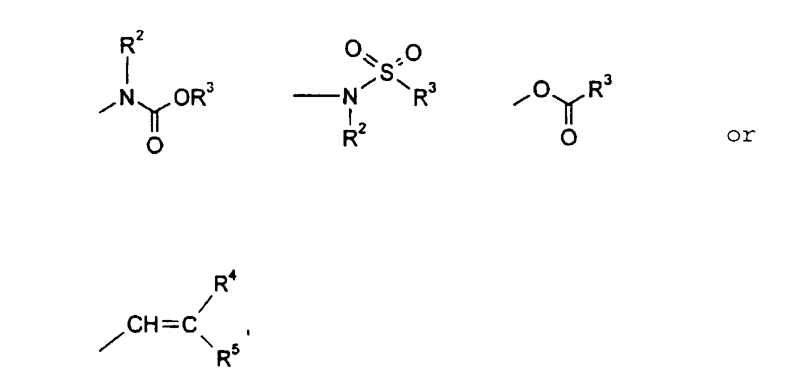
R¹ represents hydrogen or C₁-C₈-alkyl,
R² represents in each case optionally fluorine-, chlorine-, bromine-substituted C₁-C₈-alkyl, C₃-C₄-alkenyl, C₃-C₄-alkinyl, cyclopropyl, cyclopentyl, cyclohexyl, C₁-C₆-alkoxy-C₂-C₄-alkyl, C₁-C₆-alkylthio-C₂-C₄-alkyl, or represents phenyl or benzyl each of which is optionally substituted by fluorine, chlorine, bromine, nitro, cyano, methylthio, ethylthio, methoxy, ethoxy, trifluoromethylthio, trifluoromethoxy, methyl, ethyl, trifluoromethyl,
R³ represents in each case optionally fluorine-, chlorine-, bromine-substituted C₁-C₈-alkyl, or phenyl or benzyl which is substituted by fluorine, chlorine, C₁-C₂-alkyl or C₁-C₂-alkoxy,
R⁴ represents hydrogen, fluorine, chlorine, optionally fluorine- or chlorine-substituted C₁-C₄-alkyl, or phenyl which is substituted by fluorine, chlorine, methyl, ethyl, methoxy, ethoxy, trifluoromethyl, difluoromethoxy, trifluoromethoxy, cyano or nitro, and
R⁵ represents hydrogen, fluorine, chlorine or optionally fluorine- or chlorine-substituted methyl, ethyl, propyl or isopropyl,
and the enantiomerically pure forms of compounds of the formula (I).

5. Process for the preparation of the substituted aryl keto-enolic heterocycles of the formula (I) according to Claim 1, characterized in that (A), in order to obtain compounds of the formula (Ia) 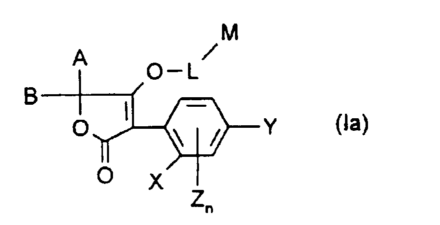 in which
A, B, L, M, X, Y, Z and n have the meaning given in Claim 1,
compounds of the formula (IIa) 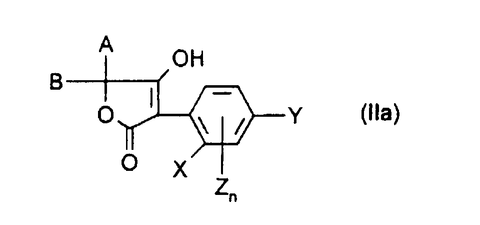 in which
A, B, X, Y, Z and n have the meaning given above are reacted with compounds of the formula (III)
G-L-M (III)
in which
L and M have the meaning given above
and
G represents a leaving group,
in the presence of a diluent and in the presence of a base,
(B)in order to obtain compounds of the formula (Ib) 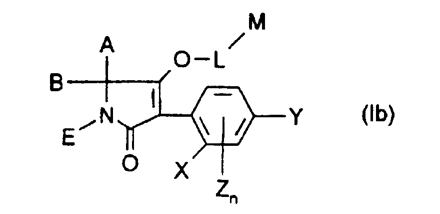 in which
A, B, E, L, M, X, Y, Z and n have the meaning given above
compounds of the formula (IIb) 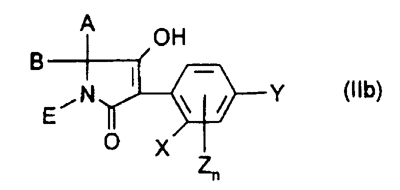 in which
A, B, E, X, Y, Z and n have the meaning given above are reacted with compounds of the formula (III)
G-L-M (III)
in which
L and M have the meaning given above
and
G represents a leaving group
in the presence of a diluent and in the presence of a base, or
(C)in order to obtain compounds of the formula (Ic) 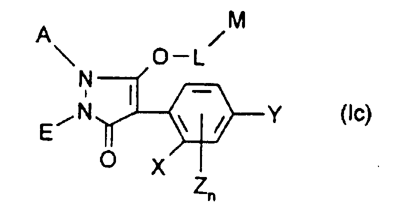 in which
A, E, L, M, X, Y, Z and n have the meaning given above
compounds of the formula (IIc) 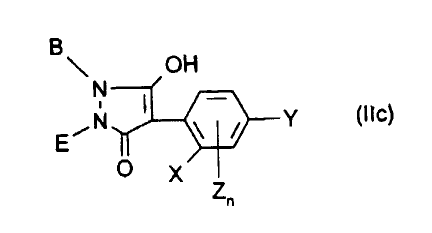 in which
B, E, X, Y, Z and n have the meaning given above are reacted with compounds of the formula (III)
G-L-M (III)
in which
L and M have the meaning given above
and
G represents a leaving group.
in the presence of a diluent and in the presence of a base.

6. Pesticide compositions, characterized by a content of at least one compound of the formula (I) according to Claim 1.

7. Arthropodicidal, fungicidal and herbicidal compositions, characterized by a content of at least one compound of the formula (I) according to Claim 1.

8. Use of compounds of the formula (I) according to Claim 1 for combating pests in plant protection, the domestic sector, the hygiene sector and in the protection of stored products.

9. Use of compounds of the formula (I) according to Claim 1 for combating arthropods, phytopatogenic fungi and unwanted plant growth in plant protection, the domestic sector, the hygiene sector and in the protection of stored products.

10. Method of combating pests in plant protection, the household sector, the hygiene sector and in the protection of stored products, characterized in that compounds of the formula (I) according to Claim 1 are caused to act on the pests and/or their habitat.

11. Process for the production of pesticide compositions, characterized in that compounds of the formula (I) according to Claim 1 are mixed with extenders and/or surface-active agents.

## Revendications

1. Composés hétérocycliques aryl-ceto-énoliques substitués répondant à la formule (I) 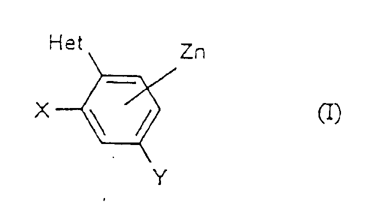 dans laquelle
X représente un groupe alkyle, un atome d'halogène ou un groupe alcoxy,
Y représente un atome d'hydrogène, un groupe alkyle, un atome d'halogène, un groupe alcoxy ou un groupe halogénalkyle,
Z représente un groupe alkyle, un atome d'halogène ou un groupe alcoxy,
n représente un nombre égal à 0, 1, 2 ou 3,
Het représente un groupe hétérocyclique choisi parmi la série comprenant 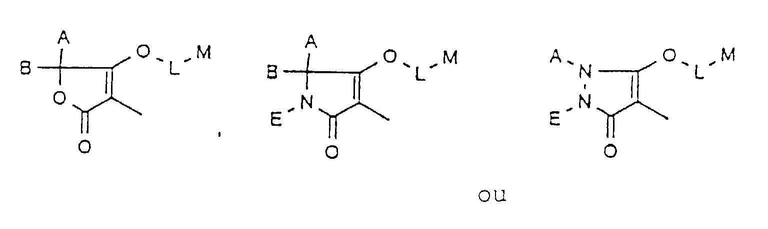
A et B peuvent être identiques ou différents et représentent un atome d'hydrogène; un groupe alkyle, un groupe alcényle, un groupe alcoxyalkyle, un groupe alkylthioalkyle, chacun de ces groupes portant le cas échéant un ou plusieurs substituants halogéno identiques ou différents; un groupe cycloalkyle le cas échéant interrompu par au moins un hétéroatome; ou encore un groupe aryle, un groupe aralkyle ou un groupe hétaryle, chacun de ces groupes portant le cas échéant un ou plusieurs substituants identiques ou différents halogéno, alkyle, halogénalkyle, alcoxy, halogénalcoxy ou nitro,
ou dans lesquels
A et B forment ensemble avec l'atome de carbone auquel ils sont liés, un cycle saturé ou insaturé, le cas échéant interrompu par au moins un hétéroatome et le cas échéant substitué,
E représente un atome d'hydrogène; un groupe alkyle, un groupe alcényle, un groupe alcoxyalkyle, un groupe alkylthioalkyle, chacun de ces groupes portant le cas échéant un ou plusieurs substituants halogéno identiques ou différents; un groupe cycloalkyle le cas échéant interrompu par au moins un hétéroatome; ou encore un groupe aryle, un groupe aralkyle ou un groupe hétaryle, chacun de ces groupes portant le cas échéant un ou plusieurs substituants identiques ou différents halogéno, alkyle, halogénalkyle, alcoxy, halogénalcoxy ou nitro,
ou dans lesquels
A et E forment ensemble avec les atomes auxquels ils sont liés, un cycle, un bicycle ou un tricycle saturé ou insaturé, le cas échéant interrompu par au moins un hétéroatome et le cas échéant substitué,
L représente un groupe alcanediyle,
M représente un des groupements ci-après: 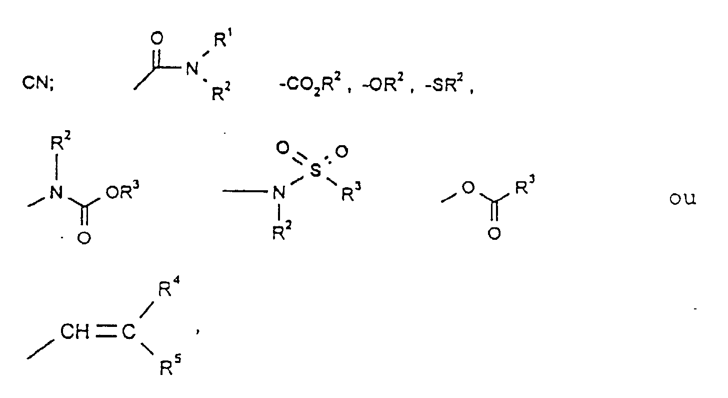
dans lesquels
R¹ représente un atome d'hydrogène ou un groupe alkyle,
R² représente un groupe alkyle, un groupe alcényle, un groupe alcynyle, un groupe cycloalkyle, un groupe alcoxyalkyle, un groupe alkylthioalkyle, un groupe aryle ou un groupe aralkyle, chacun de ces groupes étant le cas échéant substitué,
R³ représente un groupe alkyle, un groupe aryle ou un groupe aralkyle, chacun de ces groupes étant le cas échéant substitué,
R⁴ représente un atome d'hydrogène, un atome d'halogène; un groupe alkyle ou un groupe phényle, chacun de ces groupes étant le cas échéant substitué, et
R⁵ représente un atome d'hydrogène, un atome d'halogène ou un groupe alkyle le cas échéant substitué,
ainsi que les formes pures des énantiomères des composés de formule (I).

2. Composés de formule (I) selon la revendication 1, dans lesquels
X représente un groupe alkyle en C₁-C₆, un atome d'halogène ou un groupe alcoxy en C₁-C₆,
Y représente un atome d'hydrogène, un groupe alkyle en C₁-C₆, un atome d'halogène, un groupe alcoxy en C₁-C₆ ou un groupe halogénalkyle en C₁-C₃,
Z représente un groupe alkyle en C₁-C₆, un atome d'halogène ou un groupe alcoxy en C₁-C₆,
n représente un nombre de 0 à 3,
Het représente un groupe hétérocyclique choisi parmi la série comprenant 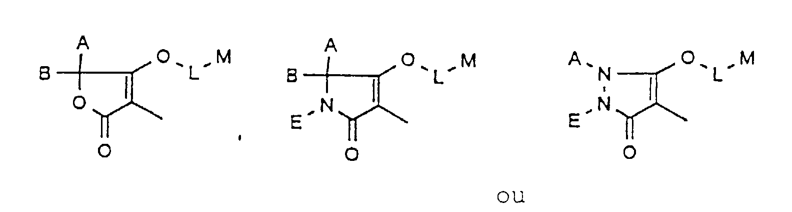
dans lesquels
A et B sont identiques ou différents et représentent un atome d'hydrogène; ou représentent un groupe alkyle en C₁-C₁₂, un groupe alcényle en C₃-C₈, un groupe alcoxy(en C₁-C₁₀) alkyle en C₂-C₈, un groupe polyalcoxy(en C₁-C₈)alkyle en C₂-C₈, un groupe alkyl(en C₁-C₁₀)thioalkyle en C₂-C₈, chacun de ces groupes étant à chaîne droite ou ramifiée et portant le cas échéant un ou plusieurs substituants halogéno identiques ou différents; un groupe cycloalkyle contenant de 3 à 8 atomes cycliques, qui peut être interrompu par un atome d'oxygène et/ou par un atome de soufre; ou encore un groupe phényle, un groupe pyrimidyle, un groupe imidazolyle, un groupe pyrazolyle, un groupe triazolyle, un groupe indolyle, un groupe thiazolyle ou un groupe phénylalkyle en C₁-C₆, chacun de ces groupes portant le cas échéant un ou plusieurs substituants identiques ou différents halogéno, alkyle en C₁-C₆, halogénalkyle en C₁-C₆, alcoxy en C₁-C₆, halogénalcoxy en C₁-C₆ ou nitro,
ou dans lesquels
A et B forment ensemble avec l'atome de carbone auquel ils sont liés, un noyau triangulaire à octogonal substitué, saturé ou insaturé, le cas échéant interrompu par un atome d'oxygène et/ou par un atome de soufre, et portant le cas échéant un ou plusieurs substituants identiques ou différents halogéno, alkyle en C₁-C₆, alcoxy en C₁-C₆, halogénalkyle en C₁-C₄, halogénalcoxy en C₁-C₄, alkyl(en C₁-C₄)thio ou phényle portant le cas échéant un ou plusieurs substituants identiques ou différents halogéno, alkyle en C₁-C₆, alcoxy en C₁-C₆,
E représente un atome d'hydrogène; ou encore un groupe alkyle en C₁-C₁₂, un groupe alcényle en C₃-C₈, un groupe alcoxy(en C₁-C₁₀)alkyle en C₂-C₈, un groupe polyalcoxy(en C₁-C₈)alkyle en C₂-C₈, un groupe alkyl(en C₁-C₁₀)thioalkyle en C₂-C₈, chacun de ces groupes étant à chaîne droite ou ramifiée et portant le cas échéant un ou plusieurs substituants halogéno identiques ou différents; un groupe cycloalkyle contenant de 3 à 8 atomes cycliques, qui peut être interrompu par un atome d'oxygène et/ou par un atome de soufre; ou encore un groupe phényle, un groupe pyrimidyle, un groupe imidazolyle, un groupe pyrazolyle, un groupe triazolyle, un groupe indolyle, un groupe thiazolyle ou un groupe phénylalkyle en C₁-C₆, chacun de ces groupes portant le cas échéant un ou plusieurs substituants identiques ou différents halogéno, alkyle en C₁-C₆, halogénalkyle en C₁-C₆, alcoxy en C₁-C₆, halogénalcoxy en C₁-C₆ ou nitro,
ou dans lesquels
A et E forment ensemble avec les atomes auxquels ils sont liés, un noyau triangulaire à octogonal substitué, saturé ou insaturé, le cas échéant interrompu par un atome d'oxygène et/ou par un atome de soufre, et portant le cas échéant un ou plusieurs substituants identiques ou différents halogéno, alkyle en C₁-C₆, alcoxy en C₁-C₆, halogénalkyle en C₁-C₄, halogénalcoxy en C₁-C₄, alkyl(en C₁-C₄)thio ou phényle portant le cas échéant un ou plusieurs substituants identiques ou différents halogéno, alkyle en C₁-C₆, alcoxy en C₁-C₆,
ou bien, pour le cas où Het dans la formule (I) représente un noyau pyrazolinone,
A et E représentent ensemble avec les deux atomes d'azote du noyau pyrazoline, un groupement portant le cas échéant un ou plusieurs substituants identiques ou différents alkyle en C₁-C₆, halogénalkyle en C₁-C₆ ou halogéno, répondant aux formules 1 à 4 indiquées ci-après: 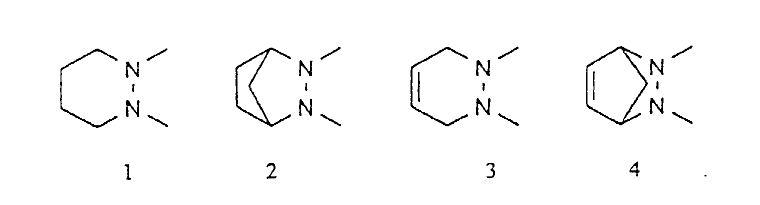
L représente un groupe alcanediyle contenant de 1 à 6 atomes de carbone,
M représente un des groupements ci-après: 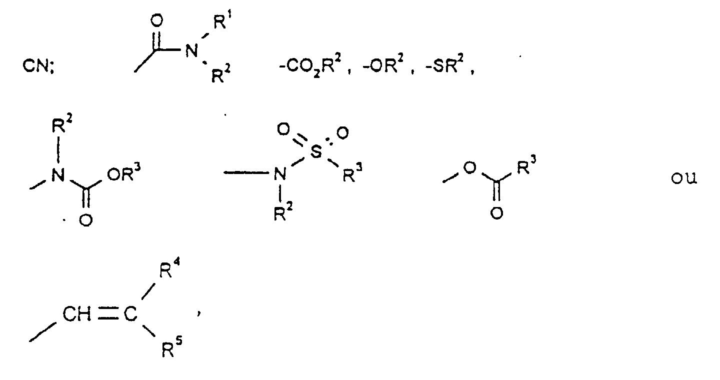
R¹ représente un atome d'hydrogène ou un groupe alkyle en C₁-C₁₂,
R² représente un groupe alkyle en C₁-C₁₂, un groupe alcényle en C₃-C₈, un groupe alcynyle en C₃-C₈, un groupe cycloalkyle en C₃-C₈, un groupe alcoxy(en C₁-C₈) alkyle en C₂-C₈, un groupe alkyl(en C₁-C₈) thioalkyle en C₂-C₈, chacun de ces groupes portant le cas échéant un ou plusieurs substituants halogéno identiques ou différents; un groupe phényle ou un groupe benzyle, chacun de ces groupes portant le cas échéant un ou plusieurs substituants identiques ou différents halogéno, nitro, cyano, alcoxy en C₁-C₆, halogénalcoxy en C₁-C₆, alkyl(en C₁-C₆)thio, halogénalkyl(en C₁-C₆)thio, alkyle en C₁-C₆ ou halogénalkyle en C₁-C₆,
R³ représente un groupe alkyle en C₁-C₁₂ portant le cas échéant un ou plusieurs substituants halogéno identiques ou différents; ou encore un groupe phényle ou un groupe benzyle, chacun de ces groupes portant un ou plusieurs substituants identiques ou différents halogéno, alkyle en C₁-C₆ ou alcoxy en C₁-C₆,
R⁴ représente un atome d'hydrogène; un atome d'halogène; un groupe alkyle en C₁-C₆ portant le cas échéant un ou plusieurs substituants halogéno identiques ou différents; ou encore un groupe phényle portant un ou plusieurs substituants identiques ou différents halogéno, alkyle en C₁-C₆, alcoxy en C₁-C₆, halogénalkyle en C₁-C₄, halogénalcoxy en C₁-C₄, cyano ou nitro, et
R⁵ représente un atome d'hydrogène, un atome d'halogène ou un groupe alkyle en C₁-C₆ portant le cas échéant un ou plusieurs substituants halogéno identiques ou différents,
ainsi que les formes pures des énantiomères des composés de formule (I).

3. Composés de formule (I) selon la revendication 1, dans lesquels
X représente un groupe alkyle en C₁-C₆, un atome de fluor, un atome de chlore, un atome de brome ou un groupe alcoxy en C₁-C₆,
Y représente un atome d'hydrogène, un groupe alkyle en C₁-C₆, un atome de fluor, un atome de chlore, un atome de brome, un groupe alcoxy en C₁-C₆ ou un groupe halogénalkyle en C₁-C₂,
Z représente un groupe alkyle en C₁-C₄, un atome de fluor, un atome de chlore, un atome de brome ou un groupe alcoxy en C₁-C₄,
n représente un nombre de 0 à 2,
Het représente un groupe hétérocyclique choisi parmi la série comprenant 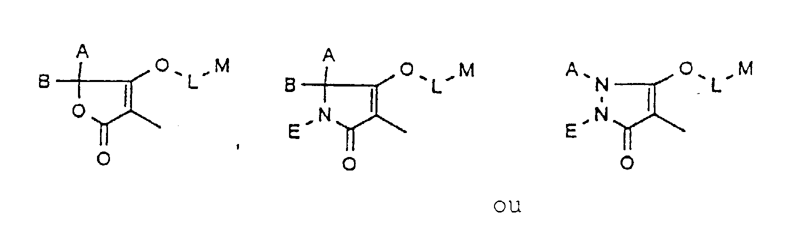
dans lesquels
A et B sont identiques ou différents et représentent un atome d'hydrogène; un groupe alkyle en C₁-C₁₀, un groupe alcényle en C₃-C₆, un groupe alcoxy(en C₁-C₈)alkyle en C₂-C₆, un groupe polyalcoxy(en C₁-C₆)alkyle en C₂-C₆, un groupe alkyl(en C₁-C₈)thioalkyle en C₂-C₆, chacun de ces groupes étant à chaîne droite ou ramifiée et portant le cas échéant un ou plusieurs substituants halogéno identiques ou différents; un groupe cycloalkyle contenant de 3 à 7 atomes cycliques, qui peut être interrompu par 1-2 atomes d'oxygène et/ou de soufre; ou représentent un groupe phényle, un groupe pyridinyle, un groupe imidazolyle, un groupe pyrazolyle, un groupe triazolyle, un groupe indolyle, un groupe thiazolyle ou un groupe phénylalkyle en C₁-C₄, chacun de ces groupes portant le cas échéant un ou plusieurs substituants identiques ou différents, halogéno, alkyle en C₁-C₄, halogénalkyle en C₁-C₄, alcoxy en C₁-C₄ ou nitro
ou dans lesquels
A et B forment ensemble avec l'atome de carbone auquel ils sont liés, un noyau triangulaire à octogonal substitué, saturé ou insaturé, le cas échéant interrompu par un atome d'oxygène et/ou par un atome de soufre, et portant le cas échéant un ou plusieurs substituants identiques ou différents halogéno, alkyle en C₁-C₅, alcoxy en C₁-C₅, halogénalkyle en C₁-C₃, halogénalcoxy en C₁-C₄, alkyl(en C₁-C₃)thio ou phényle portant le cas échéant un ou plusieurs substituants identiques ou différents halogéno, alkyle en C₁-C₄, alcoxy en C₁-C₄,
E représente un atome d'hydrogène; ou un groupe alkyle en C₁-C₁₀, un groupe alcényle en C₃-C₆, un groupe alcoxy(en C₁-C₈)alkyle en C₂-C₆, un groupe polyalcoxy(en C₁-C₆)alkyle en C₂-C₆, un groupe alkyl(en C₁-C₈)thioalkyle en C₂-C₆, chacun de ces groupes étant à chaîne droite ou ramifiée et portant le cas échéant un ou plusieurs substituants halogéno identiques ou différents; un groupe cycloalkyle contenant de 3 à 7 atomes cycliques, qui peut être interrompu par 1-2 atomes d'oxygène et/ou de soufre; ou représente un groupe phényle, un groupe pyridinyle, un groupe imidazolyle, un groupe pyrazolyle, un groupe triazolyle, un groupe indolyle, un groupe thiazolyle ou un groupe phénylalkyle en C₁-C₄, chacun de ces groupes portant le cas échéant un ou plusieurs substituants identiques ou différents halogéno, alkyle en C₁-C₄, halogénalkyle en C₁-C₄, alcoxy en C₁-C₄ ou nitro,
ou dans lesquels
A et E forment ensemble avec les atomes auxquels ils sont liés, un noyau triangulaire à octogonal substitué, saturé ou insaturé, le cas échéant interrompu par un atome d'oxygène et/ou par un atome de soufre, et portant le cas échéant un ou plusieurs substituants identiques ou différents halogéno, alkyle en C₁-C₅, alcoxy en C₁-C₅, halogénalkyle en C₁-C₃, halogénalcoxy en C₁-C₄, alkyl(en C₁-C₃)thio ou phényle portant le cas échéant un ou plusieurs substituants identiques ou différents halogéno, alkyle en C₁-C₄, alcoxy en C₁-C₄,
ou bien, pour le cas où Het dans la formule (I) représente un noyau pyrazolinone,
A et E représentent ensemble avec les deux atomes d'azote du noyau pyrazoline, un groupement portant le cas échéant un ou plusieurs substituants identiques ou différents alkyle en C₁-C₄, halogénalkyle en C₁-C₄, fluoro ou chloro, répondant aux formules 1 ou 2 indiquées ci-après: 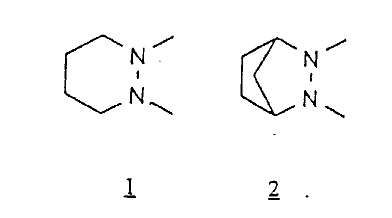
L représente un groupe alcanediyle contenant de 1 à 4 atomes de carbone,
M représente un des groupements ci-après: 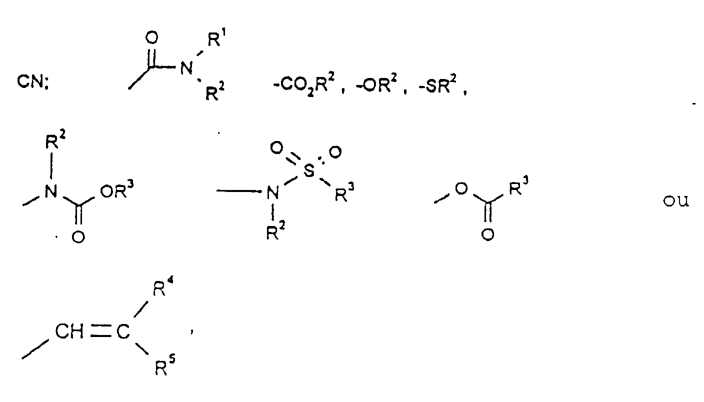
R¹ représente un atome d'hydrogène ou un groupe alkyle en C₁-C₁₀,
R² représente un groupe alkyle en C₁-C₁₀, un groupe alcényle en C₃-C₆, un groupe alcynyle en C₃-C₆, un groupe cycloalkyle en C₃-C₈, un groupe alcoxy(en C₁-C₆)alkyle en C₂-C₆, un groupe alkyl(en C₁-C₆)thioalkyle en C₂-C₆, chacun de ces groupes portant le cas échéant un ou plusieurs substituants identiques ou différents fluoro, chloro, bromo; un groupe phényle ou un groupe benzyle, chacun de ces groupes portant le cas échéant un ou plusieurs substituants identiques ou différents fluoro, chloro, bromo, nitro, cyano, alkyl(en C₁-C₄)thio, alcoxy en C₁-C₄, halogénalcoxy en C₁-C₄, halogénalcoxy(en C₁-C₄)thio, alkyle en C₁-C₄ ou halogénalkyle en C₁-C₄,
R³ représente un groupe alkyle en C₁-C₁₀ portant le cas échéant un ou plusieurs substituants identiques ou différents fluoro, chloro, bromo; ou représente un groupe phényle ou un groupe benzyle, chacun de ces groupes portant un ou plusieurs substituants identiques ou différents fluoro, chloro, alkyle en C₁-C₄ ou alcoxy en C₁-C₄,
R⁴ représente un atome d'hydrogène; un atome de fluor; un atome de chlore; un atome de brome; un groupe alkyle en C₁-C₅ portant le cas échéant un ou plusieurs substituants identiques ou différents fluoro ou chloro; ou représente un groupe phényle portant un ou plusieurs substituants identiques ou différents fluoro, chloro, bromo, alkyle en C₁-C₄, alcoxy en C₁-C₄, halogénalkyle en C₁-C₂, halogénalcoxy en C₁-C₂, cyano ou nitro, et
R⁵ représente un atome d'hydrogène; un atome de fluor; un atome de chlore; un atome de brome; ou un groupe alkyle en C₁-C₄ portant le cas échéant un ou plusieurs substituants identiques ou différents fluoro ou chloro,
ainsi que les formes pures des énantiomères des composés de formule (I).

4. Composés répondant à la formule (I) selon la revendication 1, dans lesquels
X représente un groupe méthyle, un groupe éthyle, un groupe propyle, un groupe i-propyle, un atome de fluor, un atome de chlore, un atome de brome ou un groupe méthoxy,
Y représente un atome d'hydrogène, un groupe méthyle, un groupe éthyle, un groupe propyle, un groupe i-propyle, un groupe butyle, un groupe i-butyle, un groupe tert.-butyle, un atome de fluor, un atome de chlore, un atome de brome, un groupe méthoxy, un groupe éthoxy et un groupe trifluorométhyle,
Z représente un groupe méthyle, un groupe éthyle, un groupe propyle, un groupe i-propyle, un groupe butyle, un groupe i-butyle, un groupe tert.-butyle, un atome de fluor, un atome de chlore, un atome de brome, un groupe méthoxy et un groupe éthoxy,
n représente 1,
Het représente un groupe hétérocyclique choisi parmi la série comprenant 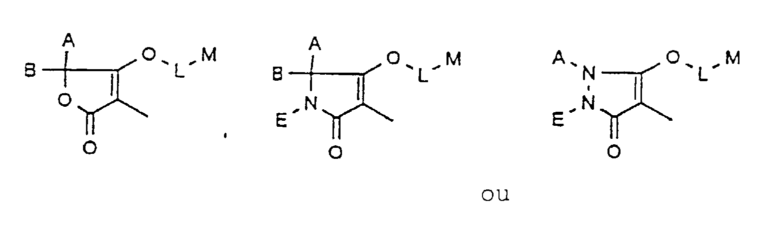
dans lesquels
A et B sont identiques ou différents et représentent un atome d'hydrogène; un groupe alkyle en C₁-C₈, un groupe alcényle en C₃-C₄, un groupe alcoxy(en C₁-C₆)alkyle en C₂-C₄, un groupe polyalcoxy(en C₁-C₄)alkyle en C₂-C₄, un groupe alkyl(en C₁-C₆)thioalkyle en C₂-C₄, chacun de ces groupes étant à chaîne droite ou ramifiée et portant le cas échéant un ou plusieurs substituants identiques ou différents fluoro ou chloro; un groupe cycloalkyle contenant de 3 à 6 atomes cycliques, qui peut être interrompu par 1-2 atomes d'oxygène et/ou de soufre; ou représentent un groupe phényle, un groupe pyridinyle, un groupe imidazolyle, un groupe indolyle ou un groupe phénylalkyle en C₁-C₃, chacun de ces groupes portant le cas échéant un ou plusieurs substituants identiques ou différents fluoro, chloro, méthyle, éthyle, propyle, isopropyle, méthoxy, éthoxy, trifluorométhyle ou nitro,
ou dans lesquels
A et B forment ensemble avec l'atome de carbone auquel ils sont liés, un noyau triangulaire à octogonal substitué, saturé ou insaturé, le cas échéant interrompu par un atome d'oxygène et/ou par un atome de soufre, et portant le cas échéant un ou plusieurs substituants identiques ou différents fluoro, chloro, alkyle en C₁-C₄, alcoxy en C₁-C₄, trifluorométhyle, alkyl(en C₁-C₂)thio ou phényle portant le cas échéant un ou plusieurs substituants identiques ou différents fluoro, chloro, méthyle, méthoxy,
E représente un atome d'hydrogène; ou un groupe alkyle en C₁-C₈, un groupe alcényle en C₃-C₄, un groupe alcoxy(en C₁-C₆)alkyle en C₂-C₄, un groupe polyalcoxy(en C₁-C₄)alkyle en C₂-C₄, un groupe alkyl(en C₁-C₆) thioalkyle en C₂-C₄, chacun de ces groupes étant à chaîne droite ou ramifiée et portant le cas échéant un ou plusieurs substituants identiques ou différents fluoro ou chloro; un groupe cycloalkyle contenant de 3 à 6 atomes cycliques, qui peut être interrompu par 1-2 atomes d'oxygène et/ou de soufre; ou représente un groupe phényle, un groupe pyridinyle, un groupe imidazolyle, un groupe indolyle ou un groupe phénylalkyle en C₁-C₃, chacun de ces groupes portant le cas échéant un ou plusieurs substituants identiques ou différents fluoro, chloro, méthyle, éthyle, propyle, isopropyle, méthoxy, éthoxy, trifluorométhyle ou nitro,
ou dans lesquels
A et E forment ensemble avec les atomes auxquels ils sont liés, un noyau triangulaire à octogonal substitué, saturé ou insaturé, le cas échéant interrompu par un atome d'oxygène et/ou par un atome de soufre, et portant le cas échéant un ou plusieurs substituants identiques ou différents fluoro, chloro, alkyle en C₁-C₄, alcoxy en C₁-C₄, trifluorométhyle, alkyl(en C₁-C₂)thio ou phényle portant le cas échéant un ou plusieurs substituants identiques ou différents fluoro, chloro, méthyle, méthoxy,
ou bien, pour le cas où Het dans la formule (I) représente un noyau pyrazolinone,
A et E représentent ensemble avec les deux atomes d'azote du noyau pyrazoline, un groupement portant le cas échéant de 1 à 3 substituants identiques ou différents méthyle, fluoro ou chloro, répondant aux formules 1 ou 2 indiquées ci-après: 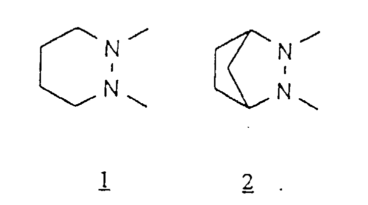
L représente un des groupements ci-après: 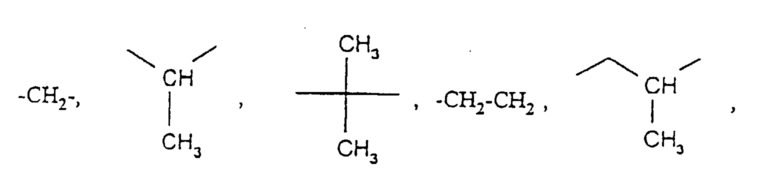
M représente un des groupements ci-après: 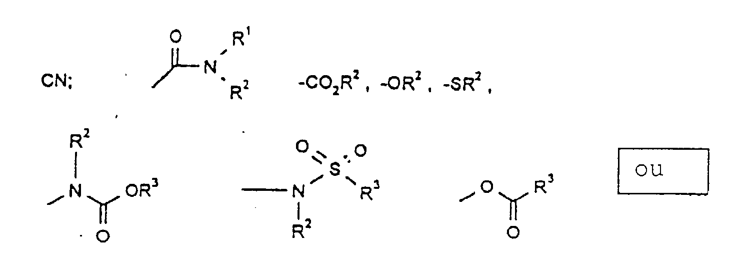 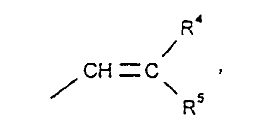
R¹ représente un atome d'hydrogène ou un groupe alkyle en C₁-C₈,
R² représente un groupe alkyle en C₁-C₈, un groupe alcényle en C₃-C₄, un groupe alcynyle en C₃-C₄, un groupe cyclopropyle, un groupe cyclopentyle, un groupe cyclohexyle, un groupe alcoxy(en C₁-C₆) alkyle en C₂-C₄, un groupe alkyl(en C₁-C₆)thioalkyle en C₂-C₄, chacun de ces groupes portant le cas échéant un ou plusieurs substituants identiques ou différents fluoro, chloro, bromo; un groupe phényle ou un groupe benzyle, chacun de ces groupes portant le cas échéant un ou plusieurs substituants identiques ou différents fluoro, chloro, bromo, nitro, cyano, méthylthio, éthylthio, méthoxy, éthoxy, trifluorométhylthio, trifluorométhoxy, méthyle, éthyle, trifluorométhyle,
R³ représente un groupe alkyle en C₁-C₈ portant le cas échéant un ou plusieurs substituants identiques ou différents fluoro, chloro, bromo; ou représente un groupe phényle ou un groupe benzyle portant un ou plusieurs substituants identiques ou différents fluoro, chloro, alkyle en C₁-C₂ ou alcoxy en C₁-C₂,
R⁴ représente un atome d'hydrogène; un atome de fluor; un atome de chlore; un groupe alkyle en C₁-C₄ portant le cas échéant un ou plusieurs substituants identiques ou différents fluoro ou chloro; ou représente un groupe phényle portant un ou plusieurs substituants identiques ou différents fluoro, chloro, méthyle, éthyle, méthoxy, éthoxy, trifluorométhyle, difluorométhoxy, trifluorométhoxy, cyano, nitro, et
R⁵ représente un atome d'hydrogène; un atome de fluor; un atome de chlore; ou représente un groupe méthyle, un groupe éthyle, un groupe propyle ou un groupe isopropyle portant le cas échéant un ou plusieurs substituants identiques ou différents fluoro ou chloro,
ainsi que les formes pures des énantiomères des composés de formule (I).

5. Procédé pour la préparation des composés hétérocycliques aryl-céto-énoliques substitués de formule (I) selon la revendication 1, caractérisé en ce qu'on fait réagir (A) pour obtenir des composés de formule (Ia) 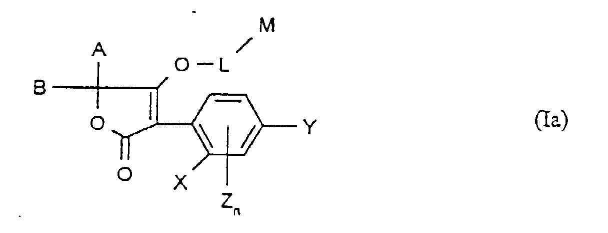 dans laquelle
A, B, L, M, X, Y, Z et n ont la signification indiquée dans la revendication 1,
des composés de formule (IIa) 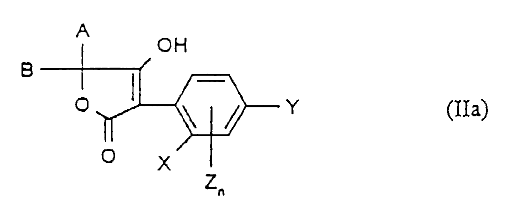 dans laquelle
A, B, X, Y, Z et n ont la signification indiquée ci-dessus,
avec des composés de formule (III)
G-L-M (III)
dans laquelle
L et M ont la signification indiquée ci-dessus,
et
G représente un groupe qui s'éloigne,
en présence d'un diluant et en présence d'une base,
(B) pour obtenir des composés de formule (Ib) 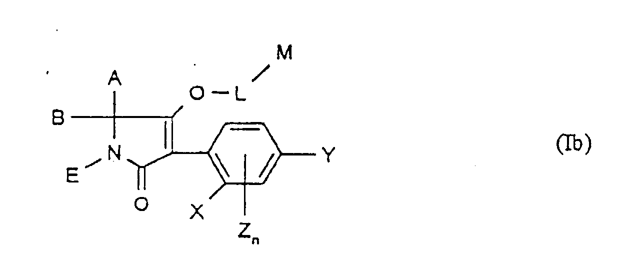 dans laquelle
A, B, E, L, M, X, Y, Z et n ont la signification indiquée ci-dessus,
des composés de formule (IIb) 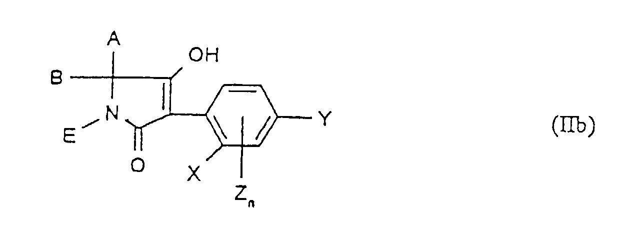 dans laquelle
A, B, E, X, Y, Z et n ont la signification indiquée ci-dessus,
avec des composés de formule (III)
G-L-M (III)
dans laquelle
L et M ont la signification indiquée ci-dessus,
et
G représente un groupe qui s'éloigne,
en présence d'un diluant et en présence d'une base,
(C) pour obtenir des composés de formule (Ic) 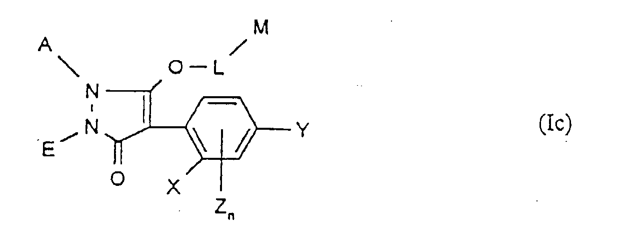 dans laquelle
A, E, L, M, X, Y, Z et n ont la signification indiquée ci-dessus,
des composés de formule (IIc) 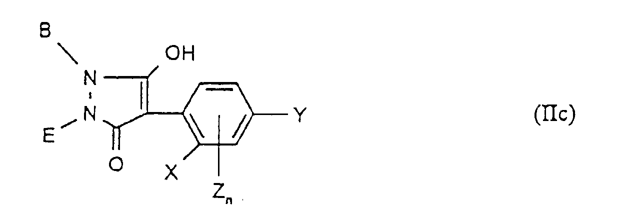 dans laquelle
B, E, X, Y, Z et n ont la signification indiquée ci-dessus,
avec des-composés de formule (III)
G-L-M (III)
dans laquelle
L et M ont la signification indiquée ci-dessus,
et
G représente un groupe qui s'éloigne,
en présence d'un diluant et en présence d'une base.

6. Agent de lutte contre les parasites, caractérisé par une teneur en au moins un composé de formule (I) selon la revendication 1.

7. Agents arthropodicides, fongicides et herbicides caractérisés par une teneur en au moins un composé de formule (I) selon la revendication 1.

8. Utilisation des composés de formule (I) selon la revendication 1 pour la lutte contre les parasites dans le domaine de protection des plantes, dans le domaine ménager, dans le domaine de l'hygiène et dans le domaine de la protection des réserves.

9. Utilisation des composés de formule (I) selon la revendication 1, pour lutter contre des arthropodes, contre des champignons phytopathogènes et contre la croissance indésirée des plantes dans le domaine de l'hygiène et dans le domaine de la protection des réserves.

10. Procédé pour lutter contre des parasites dans le domaine de l'hygiène et dans le domaine de la protection des réserves, caractérisé en ce qu'on laisse agir des composés de formule (I) selon la revendication 1 sur les parasites et/ou sur leur biotope.

11. Procédé pour la préparation d'agents de lutte contre les parasites, caractérisé en ce qu'on mélange des composés de formule (I) selon la revendication 1 avec des diluants et/ou avec des agents tensioactifs.
